# EUROPEAN PATENT SPECIFICATION

(11) **EP 2 971 137 B1**
(45) Date of publication and mention of the grant of the patent: **09.05.2018**
(21) Application number: 14765380.2
(22) Date of filing: 14.03.2014
(51) Int. Cl.: C12Q 1/68

(54) **METHODS FOR THE DETECTION OF DNA-RNA PROXIMITY IN VIVO**
VERFAHREN ZUM IN-VIVO-NACHWEIS VON DNS-RNS-NÄHE
PROCÉDÉS POUR LA DÉTECTION DE LA PROXIMITÉ D'ADN-ARN IN VIVO

(30) Priority: 15.03.2013 US 201361793783 P
(43) Date of publication of application: 20.01.2016
(73) Proprietor: The Broad Institute, Inc., Cambridge, MA 02142 (US); President and Fellows of Harvard College, Cambridge, MA 02138 (US); Massachusetts Institute of Technology, Cambridge, MA 02139 (US)
(72) Inventor: AIDEN, Erez, Lieberman, Houson, TX 77030 (US); GUTTMAN, Mitchell, West Hollywood, CA 90046 (US); STAMENOVA, Elena, Reading, MA 01867 (US); LANDER, Eric, S., Cambridge, MA 02138 (US)
(74) Representative: De Clercq, Ann G. Y.
(86) International application number: PCT/US2014/028900
(87) International publication number: WO 2014/144476

(56) References cited:
- CI CHU ET AL: "Genomic Maps of Long Noncoding RNA Occupancy Reveal Principles of RNA-Chromatin Interactions", MOLECULAR CELL, CELL PRESS, CAMBRIDGE, MA, US, vol. 44, no. 4, 31 August 2011 (2011-08-31), pages 667-678, XP028120599, ISSN: 1097-2765, DOI: 10.1016/J.MOLCEL.2011.08.027 [retrieved on 2011-09-05]
- MATTHEW J SIMMONS ET AL: "NOTCH1 inhibition in vivo results in mammary tumor regression and reduced mammary tumorsphere-forming activity in vitro", BREAST CANCER RESEARCH, CURRENT MEDICINE GROUP LTD, GB, vol. 14, no. 5, 19 September 2012 (2012-09-19), page R126, XP021130352, ISSN: 1465-5411, DOI: 10.1186/BCR3321
- CHU ET AL.: 'Genomic Maps of Long Noncoding RNA Occupancy Reveal Principles of RNA-Chromatin Interactions' MOLECULAR CELL vol. 44, 18 November 2011, pages 667 - 678, XP028120599
- ROMPPANEN, EEVA-LISA.: 'Oligonucleotide Ligation Assays for the Diagnosis of Inherited Diseases, Molecular Diagnostics' 2005, pages 31 - 40, XP008181434 Retrieved from the Internet: <URL:http://worldtracker.org/media/library/ Science / Biochemistry %20-% 20Molecular%20Biology/Molecular%20Diagnosti cs%20-%20G.%20Patrinos,%20W.% 20Ansorge%20( Elsevier ,%202005).pdf> [retrieved on 2014-06-30]
- LI ET AL.: 'Extensive Promoter-Centered Chromatin Interactions Provide a Topological Basis for Transcription Regulation' CELL vol. 148, no. ISS. 1, 20 January 2012, pages 84 - 98., XP055157332
- LU ET AL.: 'Vicinal: a method for the determination of ncRNA ends using chimeric reads from RNA-seq experiments' NUCLEIC ACIDS RESEARCH vol. 42, no. ISS. 9, 12 March 2014, pages 1 - 9, XP055284200
- RUAN, YIJUN.: 'Characterization of RNA-Chromatin Interactome by RNA-DNA Ligation and Sequening' NIH PROJECTION INFORMATION, 1R01 CA 186714-01, [Online] 26 March 2014, pages 1 - 2. Retrieved from the Internet: <URL:http://projectreporter.nih.gov/project _info_description.cfm ? aid=8673685&icde=0> [retrieved on 2014-06-30]

## Description

### FIELD OF THE DISCLOSURE

This disclosure concerns methods for the purification and/or identification of RNA and DNA molecules and/or sequence that are in close proximity in a biological system, such as a cell.

### BACKGROUND

A major goal in modern biology is defining the interactions between different biological actors *in vivo.* Over the past few decades, major advances have been made in developing methods to identify the molecular interactions with any given protein. These include methods to identify interacting proteins using co-immunoprecipitation followed by Mass-spectrometry analysis. Methods to identify interacting DNA regions using chromatin immunoprecipitation (ChIP) followed by DNA sequencing, and methods to identify interacting RNAs using RNA immunoprecipitation (RIP) or crosslinking and immunoprecipiation (CLIP). These methods have revolutionized many areas of biology and led to great advances in our understanding of transcriptional regulation, chromatin biology and epigenetics, and RNA processing and splicing.

Despite this progress in defining the interactions mediated by a specific protein, methods to define the interactions between other molecular components, such as RNA molecules and DNA molecules at a gross structural level, remain largely unexplored. Understanding the function and molecular interactions of RNA molecules and DNA molecules has lagged compared to the understanding of protein regulators primarily due to the limitations of tools to study such interactions *in vivo.* Therefore, the need exists for new and innovative methods of determining these interactions. This disclosure meets that need.

### SUMMARY OF THE DISCLOSURE

Disclosed is a method for detecting spatial proximity relationships between RNA and DNA molecules, for example in a cell, nuclear extract, a cellular milieu or system of nucleic acids of interest. The method includes: providing a sample of cells, or the nuclear extract, a cellular milieu or system of nucleic acids or interest; wherein the RNA and DNA have ends capable of joining to other DNA and RNA, respectively; joining at least one end of the RNA to the end of at least one fragmented DNA, to create at least one joined RNA-DNA hybrid molecule, wherein the join encodes the information about the proximity of the RNA and DNA in the cell; reversing the cross-linking; reverse transcribing the at least one joined RNA-DNA hybrid molecule to create least one target join DNA molecule that retains the information of the join; determining the sequence of the join, thereby detecting spatial proximity relationships between RNA and DNA molecules in a cell. In some embodiments, the RNA and/or DNA present in the sample is fragmented,

Also disclosed is a method for diagnosing a disease or condition. The method includes detecting, in a sample, one or more target joins that is indicative of a disease or condition, wherein detection of the one or more target joins that is indicative of a disease or condition diagnoses the disease or condition.

Also disclosed are joined RNA-DNA hybrid molecules, wherein the join encodes the information about the proximity of the RNA and DNA in a cell, as well as amplification products and probes for detecting such nucleic acids.

The foregoing and other features of this disclosure will become more apparent from the following detailed description of a several embodiments, which proceeds with reference to the accompanying figures.

### BRIEF DESCRIPTION OF THE FIGURES

**FIG. 1** is a schematic showing an overview of an exemplary COOL-AID procedure.

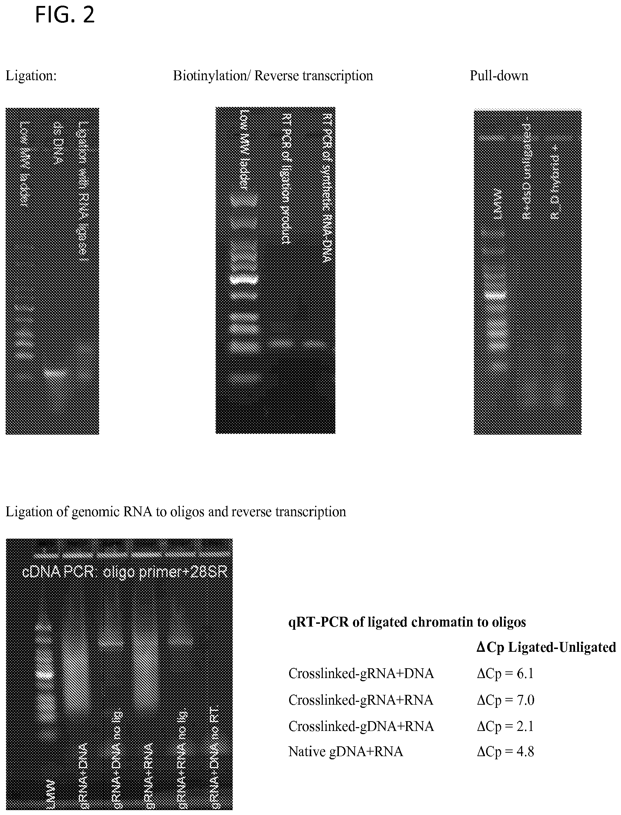

**FIG. 2** is a set of digital images of DNA gels showing the result of the methods disclosed herein. The individual gels represent the results of discrete steps in method and verify the presence of the expected complexes.

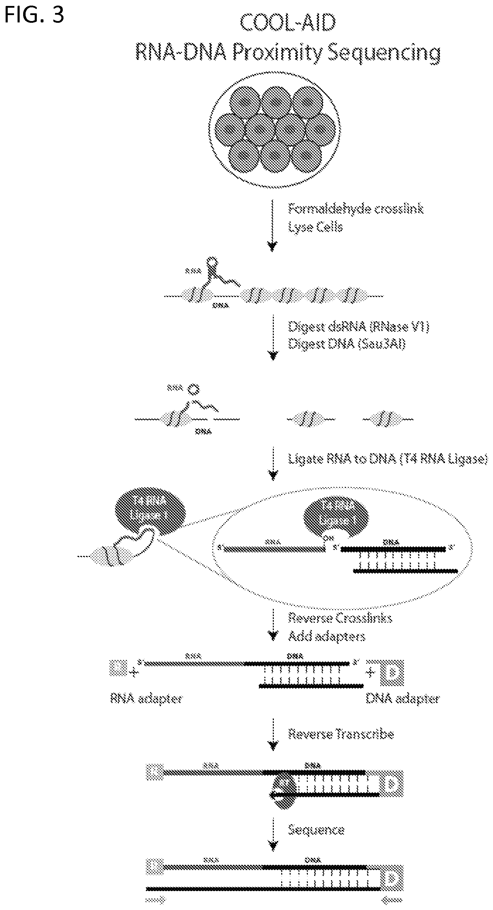

**FIG. 3** is a schematic showing an overview of an exemplary COOL-AID procedure.

### BRIEF DESCRIPTION OF THE SEQUENCES

The nucleic acid sequences shown herein are shown using standard letter abbreviations for nucleotide bases, as defined in 37 C.F.R. 1.822. Only one strand of each nucleic acid sequence is shown, but the complementary strand is understood as included by any reference to the displayed strand. The Sequence Listing is submitted as an ASCII text file in the form of the file named BROD_0200WP_ST25.txt, which was created on March 14, 2014, and is 1 kilobyte.

### DETAILED DESCRIPTION OF SEVERAL EMBODIMENTS

### Summary of Terms

Unless otherwise noted, technical terms are used according to conventional usage. Definitions of common terms in molecular biology may be found in Benjamin Lewin, Genes IX, published by Jones and Bartlet, 2008 (ISBN 0763752223); Kendrew et al. (eds.), The Encyclopedia of Molecular Biology, published by Blackwell Science Ltd., 1994 (ISBN 0632021829); and Robert A. Meyers (ed.), Molecular Biology and Biotechnology: a Comprehensive Desk Reference, published by VCH Publishers, Inc., 1995 (ISBN 9780471185710).

The singular terms "a," "an," and "the" include plural referents unless context clearly indicates otherwise. Similarly, the word "or" is intended to include "and" unless the context clearly indicates otherwise. The term "comprises" means "includes." In case of conflict, the present specification, including explanations of terms, will control.

To facilitate review of the various embodiments of this disclosure, the following explanations of specific terms are provided:
**Amplification:** To increase the number of copies of a nucleic acid molecule. The resulting amplification products are called "amplicons." Amplification of a nucleic acid molecule (such as a DNA or RNA molecule (or combination thereof) encoding a target join) refers to use of a technique that increases the number of copies of a nucleic acid molecule (including fragments).

An example of amplification is the polymerase chain reaction (PCR), in which a sample is contacted with a pair of oligonucleotide primers under conditions that allow for the hybridization of the primers to a nucleic acid template in the sample. The primers are extended under suitable conditions, dissociated from the template, re-annealed, extended, and dissociated to amplify the number of copies of the nucleic acid. This cycle can be repeated. The product of amplification can be characterized by such techniques as electrophoresis, restriction endonuclease cleavage patterns, oligonucleotide hybridization or ligation, and/or nucleic acid sequencing.

Other examples of *in vitro* amplification techniques include quantitative real-time PCR; reverse transcriptase PCR (RT-PCR); real-time PCR (rt PCR); real-time reverse transcriptase PCR (rt RT-PCR); nested PCR; strand displacement amplification (see U.S. Patent No. 5,744,311); transcription-free isothermal amplification (see U.S. Patent No. 6,033,881, repair chain reaction amplification (see WO 90/01069); ligase chain reaction amplification (see European patent publication EP-A-320 308); gap filling ligase chain reaction amplification (see U.S. Patent No. 5,427,930); coupled ligase detection and PCR (see U.S. Patent No. 6,027,889); and NASBA™ RNA transcription-free amplification (see U.S. Patent No. 6,025,134) amongst others.

**Antibody:** A polypeptide ligand comprising at least a light chain or heavy chain immunoglobulin variable region which specifically recognizes and binds an epitope of an antigen, such an epitope linked (either directly or indirectly) to a target join, that retains information about the proximity of a DNA and RNA molecule. Antibodies can include a heavy and a light chain, each of which has a variable region, termed the variable heavy (VH) region and the variable light (VL) region. Together, the VH region and the VL region are responsible for binding the antigen recognized by the antibody. This includes intact immunoglobulins and the variants and portions of them well known in the art, such as Fab' fragments, F(ab)'2 fragments, single chain Fv proteins ("scFv"), and disulfide stabilized Fv proteins ("dsFv"). The term also includes recombinant forms such as chimeric antibodies (for example, humanized murine antibodies), heteroconjugate antibodies (such as, bispecific antibodies). See also, Pierce Catalog and Handbook, 1994-1995 (Pierce Chemical Co., Rockford, IL); Kuby, Immunology, 3rd Ed., W.H. Freeman & Co., New York, 1997.

**Binding or stable binding (of an oligonucleotide):** An oligonucleotide, such as a probe for a target join, binds or stably binds to a target nucleic acid if a sufficient amount of the oligonucleotide forms base pairs or is hybridized to its target nucleic acid. Binding can be detected by either physical or functional properties.

**Binding site:** A region on a protein, DNA, or RNA to which other molecules stably bind. In one example, a binding site is the site on a RNA-DNA hybrid molecule, such as a replication competent RNA-DNA hybrid.

**Biotin-14-CTP:** A biologically active analog of cytosine-5'-triphosphate that is readily incorporated into a nucleic acid by polymerase or a reverse transcriptase. In some examples, biotin-14-CTP is incorporated into target join DNA molecule during transcription from a RNA-DNA hybrid template.

**Capture moieties:** Molecules or other substances that when attached to a nucleic acid molecule, such as a target join DNA molecule, allow for the capture of the nucleic acid molecule through interactions of the capture moiety and something that the capture moiety binds to, such as a particular surface and/or molecule, such as a specific binding molecule that is capable of specifically binding to the capture moiety.

**Contacting:** Placement in direct physical association, including both in solid or liquid form, for example contacting a sample with a crosslinking agent.

**Control:** A reference standard. A control can be a known value or range of values indicative of basal levels or amounts or present in a tissue or a cell or populations thereof. A control can also be a cellular or tissue control, for example a tissue from a non-diseased state and/or exposed to different environmental conditions. A difference between a test sample and a control can be an increase or conversely a decrease. The difference can be a qualitative difference or a quantitative difference, for example a statistically significant difference.

**Covalently linked:** Refers to a covalent linkage between atoms by the formation of a covalent bond characterized by the sharing of pairs of electrons between atoms. In one example, a covalent link is a bond between an oxygen atom and a phosphorous atom, such as phosphodiester bonds in the backbone of a nucleic acid strand. In another example, a covalent link is one between an RNA and DNA to create a replication competent RNA-DNA hybrid nucleic acid. In another example a covalent link is one between a nucleic acid and a protein or nucleic acid that has been crosslinked to by chemical means.

**Crosslinking agent:** A chemical agent or even light, which facilitates the attachment of one molecule to another molecule. Crosslinking agents can be protein-nucleic acid crosslinking agents, nucleic acid-nucleic acid crosslinking agents, and protein-protein crosslinking agents. Examples of such agents are known in the art. In some embodiments, a crosslinking agent is a reversible crosslinking agent. In some embodiments, a crosslinking agent is a non-reversible crosslinking agent.

**Complementary:** A double-stranded DNA or RNA strand consists of two complementary strands of base pairs. Complementary binding occurs when the base of one nucleic acid molecule forms a hydrogen bond to the base of another nucleic acid molecule. Normally, the base adenine (A) is complementary to thymidine (T) and uracil (U), while cytosine (C) is complementary to guanine (G). For example, the sequence 5'-ATCG-3' of one ssDNA molecule can bond to 3'-TAGC-5' of another ssDNA to form a dsDNA. In this example, the sequence 5'-ATCG-3' is the reverse complement of 3'-TAGC-5'.

Nucleic acid molecules can be complementary to each other even without complete hydrogen-bonding of all bases of each molecule. For example, hybridization with a complementary nucleic acid sequence can occur under conditions of differing stringency in which a complement will bind at some but not all nucleotide positions.

**Detect:** To determine if an agent (such as a signal or particular nucleic acid, such as a target join) is present or absent. In some examples, this can further include quantification in a sample, or a fraction of a sample, such as a particular cell or cells within a tissue.

**Detectable label:** A compound or composition that is conjugated directly or indirectly to another molecule to facilitate detection of that molecule. Specific, non-limiting examples of labels include fluorescent tags, enzymatic linkages, and radioactive isotopes. In some examples, a label is attached to an antibody or nucleic acid to facilitate detection of the molecule antibody or nucleic acid specifically binds.

**DNA sequencing:** The process of determining the nucleotide order of a given DNA molecule. Generally, the sequencing can be performed using automated Sanger sequencing (AB13730xl genome analyzer), pyrosequencing on a solid support (454 sequencing, Roche), sequencing-by-synthesis with reversible terminations (ILLUMINA® Genome Analyzer), sequencing-by-ligation (ABI SOLiD®) or sequencing-by-synthesis with virtual terminators (HELISCOPE®).

In some embodiments, DNA sequencing is performed using a chain termination method developed by Frederick Sanger, and thus termed "Sanger based sequencing" or "SBS." This technique uses sequence-specific termination of a DNA synthesis reaction using modified nucleotide substrates. Extension is initiated at a specific site on the template DNA by using a short oligonucleotide primer complementary to the template at that region. The oligonucleotide primer is extended using DNA polymerase in the presence of the four deoxynucleotide bases (DNA building blocks), along with a low concentration of a chain terminating nucleotide (most commonly a di-deoxynucleotide). Limited incorporation of the chain terminating nucleotide by the DNA polymerase results in a series of related DNA fragments that are terminated only at positions where that particular nucleotide is present. The fragments are then size-separated by electrophoresis a polyacrylamide gel, or in a narrow glass tube (capillary) filled with a viscous polymer. An alternative to using a labeled primer is to use labeled terminators instead; this method is commonly called "dye terminator sequencing."

"Pyrosequencing" is an array-based method, which has been commercialized by 454 Life Sciences. In some embodiments of the array-based methods, single-stranded DNA is annealed to beads and amplified via EmPCR®. These DNA-bound beads are then placed into wells on a fiber-optic chip along with enzymes that produce light in the presence of ATP. When free nucleotides are washed over this chip, light is produced as the PCR amplification occurs and ATP is generated when nucleotides join with their complementary base pairs. Addition of one (or more) nucleotide(s) results in a reaction that generates a light signal that is recorded, such as by the charge coupled device (CCD) camera, within the instrument. The signal strength is proportional to the number of nucleotides, for example, homopolymer stretches, incorporated in a single nucleotide flow.

**Fluorophore:** A chemical compound, which when excited by exposure to a particular stimulus such as a defined wavelength of light, emits light (fluoresces), for example at a different wavelength (such as a longer wavelength of light).

Fluorophores are part of the larger class of luminescent compounds. Luminescent compounds include chemiluminescent molecules, which do not require a particular wavelength of light to luminesce, but rather use a chemical source of energy. Therefore, the use of chemiluminescent molecules (such as aequorin) eliminates the need for an external source of electromagnetic radiation, such as a laser.

Examples of particular fluorophores that can be used in the probes disclosed herein are provided in U.S. Patent No. 5,866,366 to Nazarenko et al., such as 4-acetamido-4'-isothiocyanatostilbene-2,2'disulfonic acid, acridine and derivatives such as acridine and acridine isothiocyanate, 5-(2'-aminoethyl)aminonaphthalene-1-sulfonic acid (EDANS), 4-amino-N-[3-vinylsulfonyl)phenyl]naphthalimide-3,5 disulfonate (Lucifer Yellow VS), N-(4-anilino-1-naphthyl)maleimide, anthranilamide, Brilliant Yellow, coumarin and derivatives such as coumarin, 7-amino-4-methylcoumarin (AMC, Coumarin 120), 7-amino-4-trifluoromethylcoumarin (Coumaran 151); cyanosine; 4',6-diaminidino-2-phenylindole (DAPI); 5', 5"-dibromopyrogallol-sulfonephthalein (Bromopyrogallol Red); 7-diethylamino-3-(4'-isothiocyanatophenyl)-4-methylcoumarin; diethylenetriamine pentaacetate; 4,4'-diisothiocyanatodihydro-stilbene-2,2'-disulfonic acid; 4,4'-diisothiocyanatostilbene-2,2'-disulfonic acid; 5-[dimethylamino]naphthalene-1-sulfonyl chloride (DNS, dansyl chloride); 4-dimethylaminophenylazophenyl-4'-isothiocyanate (DABITC); eosin and derivatives such as eosin and eosin isothiocyanate; erythrosin and derivatives such as erythrosin B and erythrosin isothiocyanate; ethidium; fluorescein and derivatives such as 5-carboxyfluorescein (FAM), 5-(4,6-dichlorotriazin-2-yl)aminofluorescein (DTAF), 27'-dimethoxy-4'5'-dichloro-6-carboxyfluorescein (JOE), fluorescein, fluorescein isothiocyanate (FITC), and QFITC (XRITC); fluorescamine; IR144; IR1446; Malachite Green isothiocyanate; 4-methylumbelliferone; ortho cresolphthalein; nitrotyrosine; pararosaniline; Phenol Red; B-phycoerythrin; o-phthaldialdehyde; pyrene and derivatives such as pyrene, pyrene butyrate and succinimidyl 1-pyrene butyrate; Reactive Red 4 (Cibacron™. Brilliant Red 3B-A); rhodamine and derivatives such as 6-carboxy-X-rhodamine (ROX), 6-carboxyrhodamine (R6G), lissamine rhodamine B sulfonyl chloride, rhodamine (Rhod), rhodamine B, rhodamine 123, rhodamine X isothiocyanate, sulforhodamine B, sulforhodamine 101 and sulfonyl chloride derivative of sulforhodamine 101 (Texas Red); N,N,N',N'-tetramethyl-6-carboxyrhodamine (TAMRA); tetramethyl rhodamine; tetramethyl rhodamine isothiocyanate (TRITC); riboflavin; rosolic acid and terbium chelate derivatives; LightCycler Red 640; Cy5.5; and Cy56-carboxyfluorescein; 5-carboxyfluorescein (5-FAM); boron dipyrromethene difluoride (BODIPY); N,N,N',N'-tetramethyl-6-carboxyrhodamine (TAMRA); acridine, stilbene, -6-carboxy-fluorescein (HEX), TET (Tetramethyl fluorescein), 6-carboxy-X-rhodamine (ROX), Texas Red, 2',7'-dimethoxy-4',5'-dichloro-6-carboxyfluorescein (JOE), Cy3, Cy5, VIC® (Applied Biosystems), LC Red 640, LC Red 705, Yakima yellow amongst others.

**High throughput technique:** Through a combination of robotics, data processing and control software, liquid handling devices, and detectors, high throughput techniques allows the rapid screening of potential reagents, conditions, or targets in a short period of time, for example in less than 24, less than 12, less than 6 hours, or even less than 1 hour.

**Hybridization:** Oligonucleotides and their analogs hybridize by hydrogen bonding, which includes Watson-Crick, Hoogsteen or reversed Hoogsteen hydrogen bonding, between complementary bases. Generally, nucleic acid consists of nitrogenous bases that are either pyrimidines (cytosine (C), uracil (U), and thymine (T)) or purines (adenine (A) and guanine (G)). These nitrogenous bases form hydrogen bonds between a pyrimidine and a purine, and the bonding of the pyrimidine to the purine is referred to as "base pairing." More specifically, A will hydrogen bond to T or U, and G will bond to C. "Complementary" refers to the base pairing that occurs between two distinct nucleic acid sequences or two distinct regions of the same nucleic acid sequence.

"Specifically hybridizable" and "specifically complementary" are terms that indicate a sufficient degree of complementarity such that stable and specific binding occurs between the oligonucleotide (or it's analog) and the DNA, RNA, and or DNA-RNA hybrid target. The oligonucleotide or oligonucleotide analog need not be 100% complementary to its target sequence to be specifically hybridizable. An oligonucleotide or analog is specifically hybridizable when there is a sufficient degree of complementarity to avoid non-specific binding of the oligonucleotide or analog to non-target sequences under conditions where specific binding is desired. Such binding is referred to as specific hybridization.

**Isolated:** An "isolated" biological component (such as a protein, a nucleic acid probe, or target join described herein) has been substantially separated or purified away from other biological components in the cell of the organism in which the component naturally occurs, for example, extra-chromatin DNA and RNA, proteins and organelles. Nucleic acids and proteins that have been "isolated" include nucleic acids and proteins purified by standard purification methods. The term also embraces nucleic acids and proteins prepared by recombinant expression in a host cell as well as chemically synthesized nucleic acids. It is understood that the term "isolated" does not imply that the biological component is free of trace contamination, and can include nucleic acid molecules that are at least 50% isolated, such as at least 75%, 80%, 90%, 95%, 98%, 99%, or even 100% isolated.

**Nucleic acid (molecule or sequence):** A deoxyribonucleotide, ribonucleotide or deoxyribonucleotide-ribonucleotide polymer including without limitation, cDNA, mRNA, genomic DNA, and synthetic (such as chemically synthesized) DNA or RNA or hybrids thereof. The nucleic acid can be double-stranded (ds) or single-stranded (ss). Where single-stranded, the nucleic acid can be the sense strand or the antisense strand. Nucleic acids can include natural nucleotides (such as A, T/U, C, and G), and can also include analogs of natural nucleotides, such as labeled nucleotides.

The major nucleotides of DNA are deoxyadenosine 5 '-triphosphate (dATP or A), deoxyguanosine 5 '-triphosphate (dGTP or G), deoxycytidine 5 '-triphosphate (dCTP or C) and deoxythymidine 5 '-triphosphate (dTTP or T). The major nucleotides of RNA are adenosine 5 '-triphosphate (ATP or A), guanosine 5'-triphosphate (GTP or G), cytidine 5 '-triphosphate (CTP or C) and uridine 5'-triphosphate (UTP or U).
Nucleotides include those nucleotides containing modified bases, modified sugar moieties, and modified phosphate backbones, for example as described in U.S. Patent No. 5,866,336 to Nazarenko et al.

Examples of modified base moieties which can be used to modify nucleotides at any position on its structure include, but are not limited to: 5-fluorouracil, 5-bromouracil, 5-chlorouracil, 5-iodouracil, hypoxanthine, xanthine, acetylcytosine, 5-(carboxyhydroxylmethyl) uracil, 5-carboxymethylaminomethyl-2-thiouridine, 5-carboxymethylaminomethyluracil, dihydrouracil, beta-D-galactosylqueosine, inosine, N∼6-sopentenyladenine, 1 -methylguanine, 1-methylinosine, 2,2-dimethylguanine, 2-methyladenine, 2-methylguanine, 3-methylcytosine, 5 -methyl cytosine, N6-adenine, 7-methylguanine, 5-methylaminomethyluracil, methoxyarninomethyl-2-thiouracil, beta-D-mannosylqueosine, 5'-methoxycarboxymethyluracil, 5-methoxyuracil, 2-methylthio-N6-isopentenyladenine, uracil-5-oxyacetic acid, pseudouracil, queosine, 2-thiocytosine, 5-methyl-2-thiouracil, 2-thiouracil, 4-thiouracil, 5-methyluracil, uracil-5-oxyacetic acid methylester, uracil-S-oxyacetic acid, 5-methyl-2-thiouracil, 3-(3-amino-3-N-2-carboxypropyl) uracil, 2,6-diaminopurine and biotinylated analogs, amongst others.

Examples of modified sugar moieties which may be used to modify nucleotides at any position on its structure include, but are not limited to arabinose, 2-fluoroarabinose, xylose, and hexose, or a modified component of the phosphate backbone, such as phosphorothioate, a phosphorodithioate, a phosphoramidothioate, a phosphoramidate, a phosphordiamidate, a methylphosphonate, an alkyl phosphotriester, or a formacetal or analog thereof.

**Primers:** Short nucleic acid molecules, such as a DNA oligonucleotide, which can be annealed to a complementary target nucleic acid molecule by nucleic acid hybridization to form a hybrid between the primer and the target nucleic acid strand. A primer can be extended along the target nucleic acid molecule by a polymerase enzyme. Therefore, primers can be used to amplify a target nucleic acid molecule, wherein the sequence of the primer is specific for the target nucleic acid molecule, for example so that the primer will hybridize to the target nucleic acid molecule under very high stringency hybridization conditions.

The specificity of a primer increases with its length. Thus, for example, a primer that includes 30 consecutive nucleotides will anneal to a target sequence with a higher specificity than a corresponding primer of only 15 nucleotides. Thus, to obtain greater specificity, probes and primers can be selected that include at least 5, 10, 15, 20, 25, 30, 35, 40, 45, 50 or more consecutive nucleotides.

In particular examples, a primer is at least 15 nucleotides in length, such as at least 5 contiguous nucleotides complementary to a target nucleic acid molecule. Particular lengths of primers that can be used to practice the methods of the present disclosure include primers having at least 5, at least 10, at least 15, at least 16, at least 17, at least 18, at least 19, at least 20, at least 21, at least 22, at least 23, at least 24, at least 25, at least 26, at least 27, at least 28, at least 29, at least 30, at least 31, at least 32, at least 33, at least 34, at least 35, at least 36, at least 37, at least 38, at least 39, at least 40, at least 45, at least 50, or more contiguous nucleotides complementary to the target nucleic acid molecule to be amplified, such as a primer of 5-60 nucleotides, 15-50 nucleotides, 15-30 nucleotides or greater.

Primer pairs can be used for amplification of a nucleic acid sequence, for example, by PCR, or other nucleic-acid amplification methods known in the art. An "upstream" or "forward" primer is a primer 5' to a reference point on a nucleic acid sequence. A "downstream" or "reverse" primer is a primer 3' to a reference point on a nucleic acid sequence. In general, at least one forward and one reverse primer are included in an amplification reaction. PCR primer pairs can be derived from a known sequence, for example, by using computer programs intended for that purpose such as Primer (Version 0.5, © 1991, Whitehead Institute for Biomedical Research, Cambridge, MA).

Methods for preparing and using primers are described in, for example, Sambrook et al. (1989) Molecular Cloning: A Laboratory Manual, Cold Spring Harbor, New York; Ausubel et al. (1987) Current Protocols in Molecular Biology, Greene Publ. Assoc. & Wiley-Intersciences.

**Probe:** A probe comprises an isolated nucleic acid capable of hybridizing to a target nucleic acid (such as an target join). A detectable label or reporter molecule can be attached to a probe. Typical labels include radioactive isotopes, enzyme substrates, co-factors, ligands, chemiluminescent or fluorescent agents, haptens, and enzymes.

Methods for labeling and guidance in the choice of labels appropriate for various purposes are discussed, for example, in Sambrook et al., Molecular Cloning: A Laboratory Manual, Cold Spring Harbor Laboratory Press (1989) and Ausubel et al., Current Protocols in Molecular Biology, Greene Publishing Associates and Wiley-Intersciences (1987).

Probes are generally at least 5 nucleotides in length, such as at least 10, at least 20, at least 21, at least 22, at least 23, at least 24, at least 25, at least 26, at least 27, at least 28, at least 29, at least 30, at least 31, at least 32, at least 33, at least 34, at least 35, at least 36, at least 37, at least 38, at least 39, at least 40, at least 41, at least 42, at least 43, at least 44, at least 45, at least 46, at least 47, at least 48, at least 49, at least 50 at least 51, at least 52, at least 53, at least 54, at least 55, at least 56, at least 57, at least 58, at least 59, at least 60, or more contiguous nucleotides complementary to the target nucleic acid molecule, such as 50-60 nucleotides, 20-50 nucleotides, 20-40 nucleotides, 20-30 nucleotides or greater.

**Polymerizing agent:** A compound capable of reacting monomer molecules (such as nucleotides) together in a chemical reaction to form linear chains or a three-dimensional network of polymer chains. A particular example of a polymerizing agent is polymerase, an enzyme which catalyzes the 5' to 3' elongation of a primer strand complementary to a nucleic acid template. Examples of polymerases that can be used to amplify a nucleic acid molecule include, but are not limited to the *E. coli* DNA polymerase I, specifically the Klenow fragment which has 3' to 5' exonuclease activity, Taq polymerase, reverse transcriptase (such as HIV-1 RT), *E. coli* RNA polymerase, and wheat germ RNA polymerase II.

The choice of polymerase is dependent on the nucleic acid to be amplified. If the template is a single-stranded DNA molecule, a DNA-directed DNA or RNA polymerase can be used; if the template is a single-stranded RNA molecule, then a reverse transcriptase (such as an RNA-directed DNA polymerase) can be used.

**Targeting splint or target join probe:** A probe that includes an isolated nucleic acid capable of hybridizing to a joined replication competent RNA-DNA hybrid molecule or DNA molecule that retains the information of the join, such as a target join.

**Target join:** Any nucleic acid present or thought to be present in a sample that the information of a join between an RNA and DNA about which information would like to be obtained, such as its presence or absence. In some embodiments, a target join of interest is an RNA-DNA hybrid. In some embodiments, a target join of interest is a DNA or RNA.

**Sample:** A sample, such as a biological sample, that includes biological materials (such as nucleic acid and proteins, for example double-stranded nucleic acid binding proteins) obtained from an organism or a part thereof, such as a plant, animal, bacteria, and the like. In particular embodiments, the biological sample is obtained from an animal subject, such as a human subject. A biological sample is any solid or fluid sample obtained from, excreted by or secreted by any living organism, including without limitation, single celled organisms, such as bacteria, yeast, protozoans, and amebas among others, multicellular organisms (such as plants or animals, including samples from a healthy or apparently healthy human subject or a human patient affected by a condition or disease to be diagnosed or investigated, such as cancer). For example, a biological sample can be a biological fluid obtained from, for example, blood, plasma, serum, urine, bile, ascites, saliva, cerebrospinal fluid, aqueous or vitreous humor, or any bodily secretion, a transudate, an exudate (for example, fluid obtained from an abscess or any other site of infection or inflammation), or fluid obtained from a joint (for example, a normal joint or a joint affected by disease, such as a rheumatoid arthritis, osteoarthritis, gout or septic arthritis). A sample can also be a sample obtained from any organ or tissue (including a biopsy or autopsy specimen, such as a tumor biopsy) or can include a cell (whether a primary cell or cultured cell) or medium conditioned by any cell, tissue or organ.

**Specific Binding Agent:** An agent that binds substantially or preferentially only to a defined target such as a protein, enzyme, polysaccharide, oligonucleotide, DNA, RNA, or a small molecule. In an example, a "capture moiety specific binding agent" is capable of binding to a capture moiety that is covalently linked to a DNA molecule that retains the information about a RNA-DNA join.

A nucleic acid-specific binding agent binds substantially only to the defined nucleic acid, such as RNA, DNA or a RNA-DNA hybrid, or to a specific region within the nucleic acid. In some embodiments a specific binding agent is a targeting splint or probe for a target join, that specifically binds to a target join of interest.

**Tissue:** A plurality of functionally related cells. A tissue can be a suspension, a semi-solid, or solid. Tissue includes cells collected from a subject such as blood, cervix, uterus, lymph nodes breast, skin, and other organs.

**Under conditions that permit binding:** A phrase used to describe any environment that permits the desired activity, for example conditions under which two or more molecules, such as nucleic acid molecules and/or protein molecules, can bind.

Suitable methods and materials for the practice or testing of this disclosure are described below. Such methods and materials are illustrative only and are not intended to be limiting. Other methods and materials similar or equivalent to those described herein can be used. For example, conventional methods well known in the art to which this disclosure pertains are described in various general and more specific references, including, for example, Sambrook et al., Molecular Cloning: A Laboratory Manual, 2d ed., Cold Spring Harbor Laboratory Press, 1989; Sambrook et al., Molecular Cloning: A Laboratory Manual, 3d ed., Cold Spring Harbor Press, 2001; Ausubel et al., Current Protocols in Molecular Biology, Greene Publishing Associates, 1992 (and Supplements to 2000); Ausubel et al., Short Protocols in Molecular Biology: A Compendium of Methods from Current Protocols in Molecular Biology, 4th ed., Wiley & Sons, 1999; Harlow and Lane, Antibodies: A Laboratory Manual, Cold Spring Harbor Laboratory Press, 1990; and Harlow and Lane, Using Antibodies: A Laboratory Manual, Cold Spring Harbor Laboratory Press, 1999. In addition, the materials, methods, and examples are illustrative only and not intended to be limiting.

### II. Description of Several Embodiments

### A. Introduction

Disclosed herein is a method for detecting spatial proximity relationships between RNA and DNA *in vivo.* The disclosed method, termed "Cross-linked Oligo-Oligo Ligation And Interaction Detection" (COOL-AID), can simultaneously map substantially all of the interactions of RNAs and DNAs in a cell, including spatial arrangements of DNA and RNA, such as but not limited to hybridized RNA and DNA. By using an RNA-DNA joining step, such as a ligation step, COOL-AID can map all contacts between RNA and DNA in a cell, such as the contact between all non-coding RNAs (ncRNAs) and chromosomal DNA locations.

### B. Methods for Detecting Spatial Proximity Relationships Between RNA and DNA

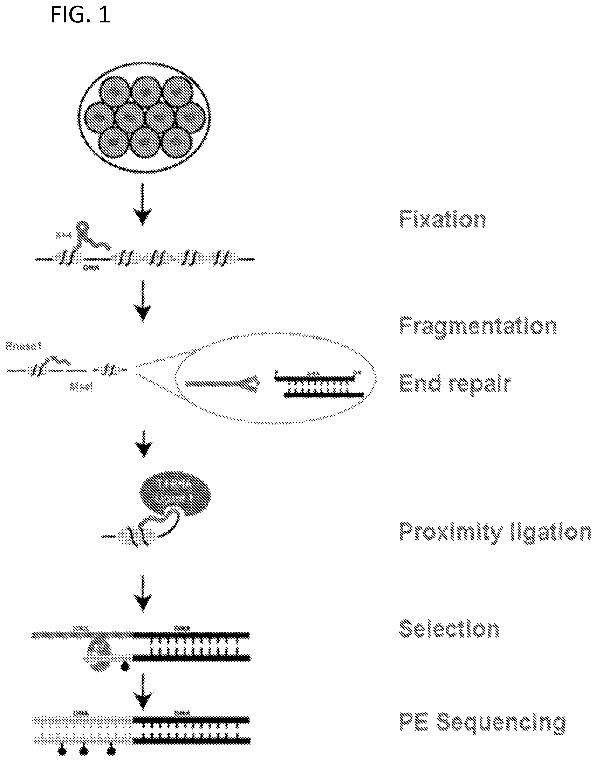

Disclosed are methods for detecting spatial proximity relationships between RNA and DNA molecules *in vivo,* for example in a cell or multiple cells. The methods include providing a sample of one or more cells, nuclear extract, cellular milieu or system of nucleic acids of interest that include nucleic acids wherein knowledge about the spatial relationships between the RNA and DNA in the sample are desired. In some examples, the sample is a sample of permeablized nuclei, mulple nuclei, isolated nuclei, synchronized cells, (such at various points in the cell cycle, for example metaphase) or acellular. In some embodiments, the nucleic acid are held in a fixed position relative to each other, such the proximity information about nucleic acids is in the samples, such as a sample of one or more cells is locked in, for example crosslinked or otherwise stabilized, for example by use of an agent such as agar that keeps the positions of the substituents in the sample from moving relative to each other in a appreciable degree. For example, a sample of cells can be treated with a crosslinker to lock in the spatial information or relationship about the molecules in the cells, such as the DNA and RNA in the cell. In another example, a sample is coated in agar. An exemplary non-limiting schematic of the method is shown in FIG. 1. With reference to FIG. 1, to detect spatial proximity relationships between RNA and DNA *in vivo,* cells are optionally treated with crosslinker (such as a protein-nucleic acid crosslinker, a nucleic acid-nucleic acid crosslinker, or a combination thereof). This treatment locks in the spatial relationships between portions of RNA and DNA in a cell, such as RNA and DNA that are hybridized or otherwise associated. Any method of fixing the nucleic acids in their positions can be used. In some embodiments, the cells are fixed, for example with a fixative, such as an aldehyde, for example formaldehyde or gluteraldehyde. In some embodiments, a sample of one or more cells is crosslinked with a corsslinker to maintain the spatial relationships in the cell. For example, a sample of cells can be treated with a crosslinker to lock in the spatial information or relationship about the molecules in the cells, such as the DNA and RNA in the cell. In other embodiments, the relative positions of the nucleic acid can be maintained with out using crosslinking agents. For example the nucleic acids can be stabilized using using spermine and spermidine (see Cullen et al., Science 261, 203 (1993), which is specifically incorporated herein by reference in its entirety). Other methods of maintaining the positional relationships of nucleic acids are known in the art.

In some embodiments, in order to create discrete portions of DNA and RNA that can be joined together in subsequent steps of the methods, the DNA and RNA present in the cells, such as crosslinked cells is fragmented. The fragmentation can be done by a variety of methods, such as enzymatic and chemical cleavage as well as mechanical shearing. For example, RNA can be fragmented using an RNAase that cuts at specific sequences of RNA, thereby yielding fragmented RNA. Suitable Rnases are commercially available, for example form New England Biolabs. One of ordinary skill in the art can choose the Rnase, with out undue experimentation. In some non-limiting examples, the Rnase is Rnase 1. Similarly, the DNA, such as chromatin bound DNA can be fragmented with one or more restriction enzymes. Suitable restriction enzymes are commercially available, for example form New England Biolabs. One of ordinary skill in the art can choose the restriction enzyme with out undue experimentation. In some non-limiting examples, the restriction enzyme is MseI. With respect to FIG. 1, because the DNA and RNA are crosslinked, either directly, or indirectly, (for example through a bridging protein, the information about spatial relationships between DNA and RNA in the cell is maintained even after the sample is fragmented. The DNA and RNA fragmentation can be done simultaneously, or stepwise. For example, the DNA can be fragmented before the RNA, or conversely, the RNA can be fragmented before the DNA. The choice of order is typically dictated by the reagents and/or methods used to fragment the DNA and RNA. One of ordinary skill in the art can choose the order based on the reagents used, with out undue experimentation. The fragmented RNA and fragmented DNA have ends capable of joining to other fragmented DNA and RNA, for example ends in close proximity. One of ordinary skill in the art will appreciate that using different fragmentation techniques, such as different enzymes with different sequence requirements, will yield different fragmentation patterns and therefore different RNA and DNA ends.

The process of fragmenting the DNA and RNA in the sample can yield ends that are capable of being joined. However, in some circumstances, the ends of the RNA and/or DNA are not competent for joining after fragmentation. Thus, is some embodiments, the ends of the RNA and/or DNA are repaired to yield ends capable of being joined. End repair can be accomplished by a variety of techniques available to one of ordinary skill in the art. It is further contemplated that the fragmentation and end repair of one type of nucleic acid, such as one of RNA or DNA, can occur prior to the digestion and/or end repair of the second type of nucleic acid.

After fragmentation and optional end repair, the RNA and DNA ends of the fragmented nucleic acids or joined together via a proximity joining step, to create a replication competent RNA-DNA hybrid. During the proximity joining step, ends of RNA are joined to ends of DNA when the ends or in close spatial proximity. Thus, with respect to the scheme shown in FIG. 1, because the DNA and RNA is still crosslinked, either directly, or indirectly, the information about spatial relationships between DNA and RNA in the cell is maintained during this proximity joining step, and substantially all of the joined RNA-DNA hybrid molecules formed at this step were in spatial proximity in the cell prior to the crosslinking step. Therefore, at this point the information about which sequences of RNA where in spatial proximity (for example bound to) DNA sequences in the cell is locked into the RNA-DNA hybrids, which can be joined, replication competent RNA-DNA hybrid molecules. While in FIG. 1 this proximity ligation step is shown using a ligase, it is contemplated that any means of joining the RNA and DNA can be used so as to create a joined replication competent RNA-DNA hybrid molecule, for example any chemical or enzymatic means. Further, it is not necessary that the ends be joined in a typical 3' 5' ligation, so long as the joined RNA and DNA is replication competent.

The RNA-DNA hybrid molecules are reverse transcribed, to create a target join DNA molecule that retains the information form the join between the ends of the fragmented RNA and DNA in the cells. In some embodiments, the crosslinks are reversed prior to reverse transcription. In some embodiments, capture moieties are introduced in the target join during reverse transcription and the target join DNA is captured molecule via the one or more capture moieties, thereby detecting spatial proximity relationships between RNA and DNA molecules in a cell.

In some embodiments, the target join DNA that includes a capture moiety is captured with a specific binding agent that specifically binds the capture moiety, for example to aid in purification. In some embodiments, the capture moiety is adsorbed or otherwise captured on a surface. In specific embodiments, the target join DNA is labeled with biotin, for instance by incorporation of biotin-14-CTP during rtPCR, allowing capture by streptavidin. Other means for labeling, capturing, and detecting nucleic acid probes include: incorporation of aminoallyl-labeled nucleotides, incorporation of sulfhydryl-labeled nucleotides, incorporation of allyl- or azide-containing nucleotides, and many other methods described in Bioconjugate Techniques (2nd Ed), Greg T. Hermanson, Elsevier (2008), which is specifically incorporated herein by reference. In some embodiments the specific binding agent is has been immobilized for example on a solid support, thereby isolating the target nucleic molecule of interest. By "solid support or carrier" is intended any support capable of binding a targeting nucleic acid. Well-known supports or carriers include glass, polystyrene, polypropylene, polyethylene, dextran, nylon, amylases, natural and modified celluloses, polyacrylamides, agrose, gabbros and magnetite. The nature of the carrier can be either soluble to some extent or insoluble for the purposes of the present disclosure. The support material may have virtually any possible structural configuration so long as the coupled molecule is capable of binding to targeting probe. Thus, the support configuration may be spherical, as in a bead, or cylindrical, as in the inside surface of a test tube, or the external surface of a rod. Alternatively, the surface may be flat such as a sheet or test strip.

After capture, these target joins are available for further analysis, for example to determine the sequences of the RNA and DNA that contributed to the information encoded by the join, which can be used to determine which DNA and RNA sequences are close in spatial proximity in the cell, for example to map specific RNA binding sites on DNA such as genomic and/or chromatin bound DNA. In some embodiments, the sequence of a the target join is determined by PCR, hybridization of a probe and/or sequencing, for example by sequencing using high-throughput paired end sequencing.

In some embodiments, determining the sequence of a target join DNA molecule includes using a probe that specifically binds to the target join DNA molecule at the site of the join. In particular embodiments, the probe specifically hybridizes to the target join both 5' and 3' of the site of the join and spans the site of the join. A probe that specifically binds to the target join DNA molecule at the site of the join can be selected based on known interactions, for example in a diagnostic setting where the presence of a particular target join, or set of target joins, has been correlated with a particular disease or condition. It is further contemplated that once a target join is known, a probe for that target join can be synthesized. It is contemplated that there may be some variation in the exact nucleotides that make up the DNA/RNA interface of the target join, thus there can be some degree of mismatch or bubble formed between the target join and the probe. In some example, a series of probes can be used to detect a target join, where the probes in the series have slightly different sequences at the site of the interface to account for slight variations in sequence.

In some embodiments, the RNA-DNA hybrids are selectively amplified. In some examples, to selectively amplify the RNA-DNA hybrids, a 3' DNA adaptor and a 5' RNA, or conversely a 5' DNA adaptor and a 3' RNA adaptor can be ligated to the ends of the molecules can be used to mark the RNA-DNA hybrids. Using primers specific for these adaptors only DNA-RNA hybrids will be amplified during an amplification procedure such as PCR. Thus, in some embodiments, an RNA adaptor nucleic acid sequence and a DNA adaptor nucleic acid sequence are ligated to the respective RNA and DNA ends of the joined replication competent RNA-DNA hybrid molecule to facilitate amplification and/or selection of the target join DNA molecule. In some embodiments, the target join DNA molecule is amplified using primers that specifically hybridize to the RNA adaptor nucleic acid sequence and the DNA adaptor nucleic acid sequence. In some examples, a capture moiety is introduced into the amplified strand of the at least one target join DNA molecule, for example to facilitate purification of target joins from other material present in the sample.

In some embodiments, the cells are lysed to release the cellular contents, for example after crosslinking. In some examples the nuclei are lysed as well, while in other examples, the nuclei are maintained intact, which can then be isolated and optionally lysed, for example using an reagent that selectively targets the nuclei or other separation technique known in the art. In some embodiments of the disclosed methods, the cells or contacted with a crosslinking agent to provide the crosslinked cells. In some examples, the cells are contacted with a protein-nucleic acid crosslinking agent, a nucleic acid-nucleic acid crosslinking agent, a protein-protein crosslinking agent or any combination thereof. By this method, the RNA and DNA present in the sample become resistant to special rearrangement and the spatial information about the DNA and RNA in the cell is maintained. In some examples, a crosslinker is a reversible crosslinker, such that the crosslinked molecules can be easily separated in subsequent steps of the method. In some examples, a crosslinker is a non-reversible crosslinker, such that the crosslinked molecules cannot be easily separated. In some examples, a crosslinker is light, such as UV light. In some examples, a cross linker is light activated. These crosslinkers include formaldehyde, disuccinimidyl glutarate, UV-254, psoralens and their derivatives such as aminomethyltrioxsalen, glutaraldehyde, ethylene glycol bis[succinimidylsuccinate], bissulfosuccinimidyl suberate, 1-Ethyl-3-[3-dimethylaminopropyl]carbodiimide (EDC) bis[sulfosuccinimidyl] suberate (BS³) and other compounds known to those skilled in the art, including those described in the Thermo Scientific Pierce Crosslinking Technical Handbook, Thermo Scientific (2009) as available on the world wide web at piercenet.com/files/1601673_Crosslink_HB_Intl.pdf.

The disclosed methods are particularly suited to monitoring disease states, such as disease state in an organism, for example a plant or an animal subject, such as a mammalian subject, for example a human subject. Certain disease states may be caused and/or characterized by the differential formation of certain target joins. For example, certain interactions may occur in a diseased cell but not in a normal cell. In other examples, certain interactions may occur in a normal cell but not in diseased cell. Thus, using the disclosed methods a profile of the interaction between a RNA and DNA *in vivo* can be correlated with a disease state. The target join profile correlated with a disease can be used as a "fingerprint" to identify and/or diagnose a disease in a cell, by virtue of having a similar "fingerprint." In addition, the profile can be used to monitor a disease state, for example to monitor the response to a therapy, disease progression and/or make treatment decisions for subjects.

The ability to obtain an interaction profile allows for the diagnosis of a disease state, for example by comparison of the profile present in a sample with the correlated with a specific disease state, wherein a similarity in profile indicates a particular disease state.

Accordingly, aspects of the disclosed methods relate to diagnosing a disease state based on target joins profile correlated with a disease state, for example cancer, or an infection, such as a viral or bacterial infection. It is understood that a diagnosis of a disease state could be made for any organism, including without limitation plants, and animals, such as humans.

Aspects of the present disclosure relate to the correlation of an environmental stress or state with an target join profile, such as a sample of cells, for example a culture of cells, can be exposed to an environmental stress, such as but not limited to heat shock, osmolarity, hypoxia, cold, oxidative stress, radiation, starvation, a chemical (for example a therapeutic agent or potential therapeutic agent) and the like. After the stress is applied, a representative sample can be subjected to analysis, for example at various time points, and compared to a control, such as a sample from an organism or cell, for example a cell from an organism, or a standard value.

In some embodiments, the disclosed methods can be used to screen chemical libraries for agents that modulate RNA-DNA interaction profiles, for example that alter the interaction profile from an abnormal one, for example correlated to a disease state to one indicative of a disease free state. By exposing cells, or fractions thereof, tissues, or even whole animals, to different members of the chemical libraries, and performing the methods described herein, different members of a chemical library can be screened for their effect on interaction profiles simultaneously in a relatively short amount of time, for example using a high throughput method.

In some embodiments, screening of test agents involves testing a combinatorial library containing a large number of potential modulator compounds. A combinatorial chemical library may be a collection of diverse chemical compounds generated by either chemical synthesis or biological synthesis, by combining a number of chemical "building blocks" such as reagents. For example, a linear combinatorial chemical library, such as a polypeptide library, is formed by combining a set of chemical building blocks (amino acids) in every possible way for a given compound length (for example the number of amino acids in a polypeptide compound). Millions of chemical compounds can be synthesized through such combinatorial mixing of chemical building blocks.

Appropriate agents can be contained in libraries, for example, synthetic or natural compounds in a combinatorial library. Numerous libraries are commercially available or can be readily produced; means for random and directed synthesis of a wide variety of organic compounds and biomolecules, including expression of randomized oligonucleotides, such as antisense oligonucleotides and oligopeptides, also are known. Alternatively, libraries of natural compounds in the form of bacterial, fungal, plant and animal extracts are available or can be readily produced. Additionally, natural or synthetically produced libraries and compounds are readily modified through conventional chemical, physical and biochemical means, and may be used to produce combinatorial libraries. Such libraries are useful for the screening of a large number of different compounds.

The compounds identified using the methods disclosed herein can serve as conventional "lead compounds" or can themselves be used as potential or actual therapeutics. In some instances, pools of candidate agents can be identified and further screened to determine which individual or subpools of agents in the collective have a desired activity.

Appropriate samples for use in the methods disclosed herein include any conventional biological sample that contains DNA and RNA, such as abtained from an organism or a part thereof, such as a plant, animal, microorganism, and the like. In particular embodiments, the biological sample is obtained from an animal subject, such as a human subject. A biological sample is any solid or fluid sample obtained from, excreted by or secreted by any living organism, including without limitation, single celled organisms, such as yeast, protozoans, and amebas among others, multicellular organisms (such as plants or animals, including samples from a healthy or apparently healthy human subject or a human patient affected by a condition or disease to be diagnosed or investigated, such as cancer). For example, a biological sample can be a biological fluid obtained from, for example, blood, plasma, serum, urine, bile, ascites, saliva, cerebrospinal fluid, aqueous or vitreous humor, or any bodily secretion, a transudate, an exudate (for example, fluid obtained from an abscess or any other site of infection or inflammation), or fluid obtained from a joint (for example, a normal joint or a joint affected by disease, such as a rheumatoid arthritis, osteoarthritis, gout or septic arthritis). A sample can also be a sample obtained from any organ or tissue (including a biopsy or autopsy specimen, such as a tumor biopsy) or can include a cell (whether a primary cell or cultured cell) or medium conditioned by any cell, tissue or organ. Exemplary samples include, without limitation, cells, cell lysates, blood smears, cytocentrifuge preparations, cytology smears, bodily fluids (*e.g.,* blood, plasma, serum, saliva, sputum, urine, bronchoalveolar lavage, semen, etc.), tissue biopsies (*e.g*., tumor biopsies), fine-needle aspirates, and/or tissue sections (*e*.*g*., cryostat tissue sections and/or paraffin-embedded tissue sections). In other examples, the sample includes circulating tumor cells (which can be identified by cell surface markers). In particular examples, samples are used directly (*e.g.,* fresh or frozen), or can be manipulated prior to use, for example, by fixation (*e.g.*, using formalin) and/or embedding in wax (such as formalin-fixed paraffin-embedded (FFPE) tissue samples). It will appreciated that any method of obtaining tissue from a subject can be utilized, and that the selection of the method used will depend upon various factors such as the type of tissue, age of the subject, or procedures available to the practitioner. Standard techniques for acquisition of such samples are available. See, for example Schluger et al., J. Exp. Med. 176:1327-33 (1992); Bigby et al., Am. Rev. Respir. Dis. 133:515-18 (1986); Kovacs et al., NEJM 318:589-93 (1988); and Ognibene et al., Am. Rev. Respir. Dis. 129:929-32 (1984).

This disclosure also provides integrated systems for high-throughput testing, or automated testing. The systems typically include a robotic armature that transfers fluid from a source to a destination, a controller that controls the robotic armature, a detector, a data storage unit that records detection, and an assay component such as a microtiter dish comprising a well having a reaction mixture for example media.

In some embodiments of the disclosed methods, determining the identity of a nucleic acid, such as a target join, includes detection by nucleic acid hybridization. Nucleic acid hybridization involves providing a probe and target nucleic acid under conditions where the probe and its complementary target can form stable hybrid duplexes through complementary base pairing. The nucleic acids that do not form hybrid duplexes are then washed away leaving the hybridized nucleic acids to be detected, typically through detection of an attached detectable label. It is generally recognized that nucleic acids are denatured by increasing the temperature or decreasing the salt concentration of the buffer containing the nucleic acids. Under low stringency conditions (*e.g*., low temperature and/or high salt) hybrid duplexes (*e.g*., DNA:DNA, RNA:RNA, or RNA:DNA) will form even where the annealed sequences are not perfectly complementary. Thus, specificity of hybridization is reduced at lower stringency. Conversely, at higher stringency (*e*.*g*., higher temperature or lower salt) successful hybridization requires fewer mismatches. One of skill in the art will appreciate that hybridization conditions can be designed to provide different degrees of stringency.

In general, there is a tradeoff between hybridization specificity (stringency) and signal intensity. Thus, in one embodiment, the wash is performed at the highest stringency that produces consistent results and that provides a signal intensity greater than approximately 10% of the background intensity. Thus, the hybridized array may be washed at successively higher stringency solutions and read between each wash. Analysis of the data sets thus produced will reveal a wash stringency above which the hybridization pattern is not appreciably altered and which provides adequate signal for the particular oligonucleotide probes of interest. In some examples, RNA is detected using Northern blotting or *in situ* hybridization (Parker & Barnes, Methods in Molecular Biology 106:247-283, 1999); RNAse protection assays (Hod, Biotechniques 13:852-4, 1992); and PCR-based methods, such as reverse transcription polymerase chain reaction (RT-PCR) (Weis et al., Trends in Genetics 8:263-4, 1992).

In one embodiment, the hybridized nucleic acids are detected by detecting one or more labels attached to the sample nucleic acids. The labels can be incorporated by any of a number of methods. In one example, the label is simultaneously incorporated during the amplification step in the preparation of the sample nucleic acids. Thus, for example, polymerase chain reaction (PCR) with labeled primers or labeled nucleotides will provide a labeled amplification product. In one embodiment, transcription amplification, as described above, using a labeled nucleotide (such as fluorescein-labeled UTP and/or CTP) incorporates a label into the transcribed nucleic acids.

Detectable labels suitable for use include any composition detectable by spectroscopic, photochemical, biochemical, immunochemical, electrical, optical or chemical means. Useful labels include biotin for staining with labeled streptavidin conjugate, magnetic beads (for example DYNABEADS™), fluorescent dyes (for example, fluorescein, Texas red, rhodamine, green fluorescent protein, and the like), radiolabels (for example, ³H, ¹²⁵I, ³⁵S, ¹⁴C, or ³²P), enzymes (for example, horseradish peroxidase, alkaline phosphatase and others commonly used in an ELISA), and colorimetric labels such as colloidal gold or colored glass or plastic (for example, polystyrene, polypropylene, latex, etc.) beads. Patents teaching the use of such labels include U.S. Patent No. 3,817,837; U.S. Patent No. 3,850,752; U.S. Patent No. 3,939,350; U.S. Patent No. 3,996,345; U.S. Patent No. 4,277,437; U.S. Patent No. 4,275,149; and U.S. Patent No. 4,366,241.

Means of detecting such labels are also well known. Thus, for example, radiolabels may be detected using photographic film or scintillation counters, fluorescent markers may be detected using a photodetector to detect emitted light. Enzymatic labels are typically detected by providing the enzyme with a substrate and detecting the reaction product produced by the action of the enzyme on the substrate, and colorimetric labels are detected by simply visualizing the colored label.

The label may be added to the target (sample) nucleic acid(s) prior to, or after, the hybridization. So-called "direct labels" are detectable labels that are directly attached to or incorporated into the target (sample) nucleic acid prior to hybridization. In contrast, so-called "indirect labels" are joined to the hybrid duplex after hybridization. Often, the indirect label is attached to a binding moiety that has been attached to the target nucleic acid prior to the hybridization. Thus, for example, the target nucleic acid may be biotinylated before the hybridization. After hybridization, an avidin-conjugated fluorophore will bind the biotin bearing hybrid duplexes providing a label that is easily detected (see Laboratory Techniques in Biochemistry and Molecular Biology, Vol. 24: Hybridization With Nucleic Acid Probes, P. Tijssen, ed. Elsevier, N.Y., 1993).

In some embodiments, the identity of a nucleic acid is determined by DNA or RNA sequencing. Generally, the sequencing can be performed using automated Sanger sequencing (AB13730xl genome analyzer), pyrosequencing on a solid support (454 sequencing, Roche), sequencing-by-synthesis with reversible terminations (ILLUMINA® Genome Analyzer), sequencing-by-ligation (ABI SOLiD®) or sequencing-by-synthesis with virtual terminators (HELISCOPE®).

Examples of information that can be obtained form the disclosed methods and the analysis of the results thereof, include without limitation, 3 dimensional genome mapping, genome assembly, one dimensional genome mapping, the use of single nucleotide polymorphisms to phase genome maps, for example to determine the patterns of chromosome inactivation, such as for analysis of genomic imprinting.

### C. Target Joins and Target Join Probes

Also disclosed are isolated joins and amplification products thereof, such as RNA, DNA or a combination thereof. An isolated join is a joined RNA-DNA hybrid molecule, wherein the join encodes the information about the proximity of the RNA and DNA in a cell, for example as formed by the methods disclosed herein. The presence of an isolated join can be correlated with a disease state or environmental condition. For example, certain disease states may be caused and/or characterized by the differential formation of certain target joins. Similarly isolated joins can be correlated to an environmental stress or state, such as but not limited to heat shock, osmolarity, hypoxia, cold, oxidative stress, radiation, starvation, a chemical (for example a therapeutic agent or potential therapeutic agent) and the like.

This disclosure also relates, to isolated nucleic acid probes that specifically bind to target joins, such as a target join indicative of a disease state or environmental condition. To recognize a target join, a probe specifically hybridizes to the target join both 5' and 3' of the site of the join and spans the site of the join. In some embodiments, the probe is labeled, such as radiolabeled, fluorescently-labeled, biotin-labeled, enzymatically-labeled, or chemically-labeled. Non-limiting examples of the probe is an RNA probe, a DNA probe, a locked nucleic acid (LNA) probe, or a hybrid RNA-DNA probe. Also disclosed are sets of probes for binding to target joins, as well as devices, such as nucleic acid arrays for detecting a target join.

In embodiments, the total length of the probe, including end linked PCR or other tags, is between about 10 nucleotides and 200 nucleotides, although longer probes are contemplated. In some embodiments, the total length of the probe, including end linked PCR or other tags, is at least about 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, 50, 51, 52, 53, 54, 55, 56, 57, 58, 59, 60, 61, 62, 63, 64, 65, 66, 67, 68, 69, 70, 71, 72, 73, 74, 75, 76, 77, 78, 79, 80, 81, 82, 83, 84, 85, 86, 87, 88, 89, 90, 91, 92, 93, 94, 95, 96, 97, 98, 99, 100, 101, 102, 103, 104, 105, 106, 107, 108, 109, 110, 111, 112, 113, 114, 115, 116, 117, 118, 119, 120, 121, 122, 123, 124, 125, 126, 127, 128, 129, 130, 131, 132, 133, 134, 135, 136, 137, 138, 139, 140, 141, 142, 143, 144, 145, 146, 147, 148, 149, 150, 151, 152, 153, 154, 155, 156, 157, 158, 159, 160, 161, 162, 163, 164, 165, 166, 167, 168, 169, 170, 171, 172, 173, 174, 175, 176, 177, 178, 179, 180, 181, 182, 183, 184, 185, 186, 187, 188, 189, 190 191, 192, 193, 194, 195, 196, 197, 198, 199 or 200.

In some embodiments the total length of the probe, including end linked PCR or other tags, is less then about 2000 nucleotides in length, such as less than about 121, 122, 123, 124, 125, 126, 127, 128, 129, 130, 131, 132, 133, 134, 135, 136, 137, 138, 139, 140, 141, 142, 143, 144, 145, 146, 147, 148, 149, 150, 151, 152, 153, 154, 155, 156, 157, 158, 159, 160, 161, 162, 163, 164, 165, 166, 167, 168, 169, 170, 171, 172, 173, 174, 175, 176, 177, 178, 179, 180, 181, 182, 183, 184, 185, 186, 187, 188, 189, 190 191, 192, 193, 194, 195, 196, 197, 198, 199, 200, 201, 202, 203, 204, 205, 206, 207, 208, 209, 210, 211, 212, 213, 214, 215, 216, 217, 218, 219, 220, 221, 222, 223, 224, 225, 226, 227, 228, 229, 230, 231, 232, 233, 234, 235, 236, 237, 238, 239, 240, 241, 242, 243, 244, 245, 246, 247, 248, 249, 250, 500, 750, 1000, 1250, 1500, 1750, 2000 nucleotides in length or even greater. In some embodiments, the total length of the probe, including end linked PCR or other tags, is between about 30 nucleotides and about 250 nucleotides, for example about 90 to about 180, about 120 to about 200, about 150 to about 220 or about 120 to about 180 nucleotides in length. In some embodiments, a set of probes is used to target a specific target join or a set of target joins.

In some embodiments, the probe is detectably labeled, either with an isotopic or non-isotopic label, alternatively the target join or amplification product thereof is labeled. Non-isotopic labels can, for instance, comprise a fluorescent or luminescent molecule, biotin, an enzyme or enzyme substrate or a chemical. Such labels are preferentially chosen such that the hybridization of the probe with target join can be detected. In some examples, the probe is labeled with a fluorophore. Examples of suitable fluorophore labels are given above. In some examples, the fluorophore is a donor fluorophore. In other examples, the fluorophore is an accepter fluorophore, such as a fluorescence quencher. In some examples, the probe includes both a donor fluorophore and an accepter fluorophore. Appropriate donor/acceptor fluorophore pairs can be selected using routine methods. In one example, the donor emission wavelength is one that can significantly excite the acceptor, thereby generating a detectable emission from the acceptor.

An array containing a plurality of heterogeneous probes for the detection of target joins are disclosed. Such arrays may be used to rapidly detect and/or identify the target joins present in a sample, for example as part of a diagnosis. Arrays are arrangements of addressable locations on a substrate, with each address containing a nucleic acid, such as a probe. In some embodiments, each address corresponds to a single type or class of nucleic acid, such as a single probe, though a particular nucleic acid may be redundantly contained at multiple addresses. A "microarray" is a miniaturized array requiring microscopic examination for detection of hybridization. Larger "macroarrays" allow each address to be recognizable by the naked human eye and, in some embodiments, a hybridization signal is detectable without additional magnification. The addresses may be labeled, keyed to a separate guide, or otherwise identified by location.

Any sample potentially containing, or even suspected of containing, a target joins may be used. A hybridization signal from an individual address on the array indicates that the probe hybridizes to a nucleotide within the sample. This system permits the simultaneous analysis of a sample by plural probes and yields information identifying the target joins contained within the sample. In alternative embodiments, the array contains target joins and the array is contacted with a sample containing a probe. In any such embodiment, either the probe or the target join may be labeled to facilitate detection of hybridization.

Within an array, each arrayed nucleic acid is addressable, such that its location may be reliably and consistently determined within the at least the two dimensions of the array surface. Thus, ordered arrays allow assignment of the location of each nucleic acid at the time it is placed within the array. Usually, an array map or key is provided to correlate each address with the appropriate nucleic acid. Ordered arrays are often arranged in a symmetrical grid pattern, but nucleic acids could be arranged in other patterns (for example, in radially distributed lines, a "spokes and wheel" pattern, or ordered clusters). Addressable arrays can be computer readable; a computer can be programmed to correlate a particular address on the array with information about the sample at that position, such as hybridization or binding data, including signal intensity. In some exemplary computer readable formats, the individual samples or molecules in the array are arranged regularly (for example, in a Cartesian grid pattern), which can be correlated to address information by a computer.

An address within the array may be of any suitable shape and size. In some embodiments, the nucleic acids are suspended in a liquid medium and contained within square or rectangular wells on the array substrate. However, the nucleic acids may be contained in regions that are essentially triangular, oval, circular, or irregular. The overall shape of the array itself also may vary, though in some embodiments it is substantially flat and rectangular or square in shape.

Examples of substrates for the phage arrays disclosed herein include glass (e.g., functionalized glass), Si, Ge, GaAs, GaP, SiO₂, SiN₄, modified silicon nitrocellulose, polyvinylidene fluoride, polystyrene, polytetrafluoroethylene, polycarbonate, nylon, fiber, or combinations thereof. Array substrates can be stiff and relatively inflexible (for example glass or a supported membrane) or flexible (such as a polymer membrane). One commercially available product line suitable for probe arrays described herein is the Microlite line of MICROTITER® plates available from Dynex Technologies UK (Middlesex, United Kingdom), such as the Microlite 1+ 96-well plate, or the 384 Microlite+ 384-well plate.

Addresses on the array should be discrete, in that hybridization signals from individual addresses can be distinguished from signals of neighboring addresses, either by the naked eye (macroarrays) or by scanning or reading by a piece of equipment or with the assistance of a microscope (microarrays).

### D. Kits

The nucleic acid probes and other reagents disclosed herein for use in the disclosed methods can be supplied in the form of a kit. In such a kit, an appropriate amount of one or more of the nucleic acid probes is provided in one or more containers or held on a substrate. A nucleic acid probe may be provided suspended in an aqueous solution or as a freeze-dried or lyophilized powder, for instance. The container(s) in which the nucleic acid(s) are supplied can be any conventional container that is capable of holding the supplied form, for instance, microfuge tubes, ampoules, or bottles. The kits can include either labeled or unlabeled nucleic acid probes for use in detection, of a target join. The amount of nucleic acid probe supplied in the kit can be any appropriate amount, and may depend on the target market to which the product is directed. A kit may contain more than one different probe, such as 2, 3, 4, 5, 6, 7, 8, 9, 10, 11, 12, 13, 14, 15, 20, 25, 50, 100, or more probes. The instructions may include directions for obtaining a sample, processing the sample, preparing the probes, and/or contacting each probe with an aliquot of the sample. In certain embodiments, the kit includes an apparatus for separating the different probes, such as individual containers (for example, microtubules) or an array substrate (such as, a 96-well or 384-well microtiter plate). In particular embodiments, the kit includes prepackaged probes, such as probes suspended in suitable medium in individual containers (for example, individually sealed EPPENDORF® tubes) or the wells of an array substrate (for example, a 96-well microtiter plate sealed with a protective plastic film). In some embodiments, kits also may include the reagents necessary to carry out methods disclosed herein. In other particular embodiments, the kit includes equipment, reagents, and instructions for the methods disclosed herein.

The following examples are provided to illustrate certain particular features and/or embodiments. These examples should not be construed to limit the invention which is defined in the appended claims to the particular features or embodiments described.

### EXAMPLES

### Example 1

The following protocol was used as a non-limiting example of the methods and compositions disclosed herein. While specific times and reagents are specified, it is contemplated that different albeit similar reagents and times and temperatures can be employed by those of ordinary skill in the art with minimal experimentation, given the guidance presented herein.

### Protocol

### Cell lysis and chromatin digestion

### 1 pellet of formaldehyde crosslinked human lymphoblastoid cells GM 12878 of 2x10⁷

### Or 2 formaldehyde crosslinked MLF pellets (2.5x10⁶ each)

1. Cells are lysed with Hi-C lysis buffer (500 µl 10 mM Tris-HCl pH8.0, 10 mM NaCl, 0.2% Igepal CA630)with added 50ul protease inhibitors and 25ul AntiRnase
2. Incubate on ice > 15 minutes
3. Lyse cells with a Dounce homogenizer, moving the pestle up and down >30 times on ice

### RNA digestion

1. Add 60ul Rnase 1 diluted (**1:250**) to each tube
2. Mix well and incubate at 37C for 10 min
3. To stop the reaction, add 10ul Speraseln to each tube and mix well
4. Add 60 ul 1%SDS to the tube and incubate for exactly 10min at 62C
5. Quench SDS with 70 ul 10% Triton X and mix well. Put on ice.
6. Spin at 2500G for 5 min
7. Remove supernatant and wash pellet with 500ul cold TLE. Spin as above
8. Resuspend pellet in 500ul 1XAntractic Phosphatase buffer.

### RNA end repair

1. To remove the 2',3' cyclic monophosphate, add:
   50 ul EDTA-free protease inhibitors
   5ul Superaseln
   50 ul NEB antractic phosphatase (NEB #M0289S), 5u/ul
2. Incubate at 37 for 20 min
3. Inactivate AP by adding 50ul 10%SDS and incubating at 62C for 5 min.
4. Quench SDS with 70ul 10% Triton X and mix well
5. Put on ice. Spin at 2500G for 5 min
6. Remove supernatant and wash pellet with 500ul cold IX Restriction Buffer. Spin as above
7. Repeat wash twice
8. Resuspend pellet in 250ul 1XNEB4.

### DNA restriction digest

1. Distribute lysate to 5 tubes (**50 ul per tube**)
2. Add **400ul NEB4; 5 ul SuperaseIn; 5ul BSA (10mg/ml) and 8 ul x 50,000u/ml of MseI**
3. Chromatin is digested overnight at 37°C while rotating

### Proximity Ligation

Prepare 5ml of 10x ligation buffer (500 mM Tris-HCl pH 7.5, 100 mM MgCl2, 100 mM DTT)

| | Stock | Volume (ml) | Final Conc. |
|---|---|---|---|
| Tris-HCl, pH 7.5 | 1M | 2.5 | 0.5 |
| MgCl₂ | 1M | 0.5 | 0.1 |
| DTT | 1M | 0.5 | 0.1 |
| Water | | 1.5 | |

1) Prepare Ligation master mix as follow:

| **Ligation mix (add in the listed order)** | **Per reaction** | **5 reactions, ml** |
|---|---|---|
| Water | 5.74 ml | 30,422 |
| 10x ligation buffer | 800 µl | 4,240 |
| DMSO | 800 µl | 4,240 |
| 10 mg/ml BSA | 80 µl | 0.424 |
| 100mM ATP | 80 µl | 0.424 |

2) Working on ice, add 7.4 ml ligation mix to each of five labeled 15 ml tubes.
3) Transfer 600ul digested chromatin mixture to a corresponding 15 ml tube.
4) For positive ligation control, add 20 µl 1U/µl T4 RNA ligase 1 to tubes 1-3.
5) For negative control of ligation, add 20 µl water to tubes 4, 5.
6) Mix by inverting the tubes and incubate all 5 tubes for 6.5 hours at 16°C.

### Cross-link reversal and purification

1) Crosslinks are reversed and protein is degraded by adding to each tube:
   80 ul of 0.5M EDTA
   80 µl 10% SDS
   30 µl 10 mg/ml proteinase K
2) Incubate at 55°C with mixing for 0.5 hours.
3) Add 800ul 5M NaCl to each tube and incubate at 68°C with mixing for 1.5-2 hours
4) Cool the reaction mixtures to room temperature and transfer them to five 50 ml conical tubes. Rinse 15ml tubes with 1ml of water or 1XTLE. Purify RNA_DNA in these tubes by adding 10 ml phenol pH 8.0 and vortexing for 2 minutes. Spin the tubes for 10 minutes at 3,500 rpm and carefully transfer as much of the aqueous phase as possible to a new 50 ml tube.
5) Repeat the extraction using phenol pH 8.0:chloroform (1:1) and precipitate the DNA 0.1x vol. 3M Na-acetate, pH 5.2 and 2.5x vol. ice cold ethanol. Mix and incubate at -80 for 1h. Spin at max speed for 30 min.
6) Resuspend each pellet in 70% ethanol and transfer to 1.5ml tube. Spin at max speed for 5 mins. Dissolve in 500ul IX TLE.
7) Another round of purification is performed by doing 2 phenol:chloroform extractions. Add 500 µl phenol pH 8.0:chloroform (1:1) and vortex for 1 minute. Centrifuge the tubes for 3 minutes at 14,000 rpm and transfer the aqueous phase to a new tube.
8) After the second extraction, precipitate the DNA by adding 0.1x volume of sodium acetate, 2x volume of 100% ethanol and incubate 30 minutes at -80 °C.
9) After spinning down the precipitated DNA, wash each pellet twice with 70% ethanol and resuspend DNA pellet in 25 µl 1x TE.
10) Pool all positive tubes together, pool all negative controls in a separate tube

### RT transcription with biotinylated dCTP

| | **onc.** | **ul stock for 10 ul library** | **Master mix for vol. of library** | | | |
|---|---|---|---|---|---|---|
| | | | **25 ul** | **50 ul** | **75 ul** | **100 ul** |
| **5X First strand buffer** | 5 X | 4 | 10 | 20.8 | 31.2 | 42 |
| **DTT** | 100 mM | 0.8 | 2 | 4.2 | 6.2 | 8.4 |
| **SuperScript III RT** | 200 U/ul | 1 | 2.5 | 5.2 | 7.8 | 10.5 |
| **dGTP, dTTP mix** | 10 mM each | 1 | 2.5 | 5.2 | 7.8 | 10.5 |
| | 10 mM | 0.2 | 0.5 | 1.0 | 1.56 | 2.1 |
| **dCTP** | 0.4 mM | 2.5 | 6.3 | 13 | 19.5 | 26.3 |
| se In | 20U/ul | 0.5 | 1.3 | 2.6 | 3.9 | 5.3 |

- Heat the RNA-DNA solution to 70C for 3 min in heating block to denature secondary structures. Place on ice for 1-5min (do not leave on ice for long time) before adding to other reagents
- Aliquot volume of master mix equal to volume of library used to 0.2 ulPCR tubes
- Add equal volume of library to corresponding tubes
- Mix well; flash spin tubes
- Incubate at RT for 10 min
- In thermal cycle
   ∘ Incubate at 55C for 1h
   ∘ Inactivate reaction by heating at 70C for 15 min. Follow by refrigeration
- Remove bio-dCTP
   ∘ Vortex a Zeba desalt column (Thermo Scientific)
   ∘ Brake the tip, place on 2ml capless tube and open screw cap one turn
   ∘ Spin 2 min at 1,000 xG at RT
   ∘ Apply 500 ul TLE on top of resin bed and spin 2 min at 1,000 xG at RT. Repeat 2x
   ∘ Place columns in new 1.5 mL tubes, place the lower part of the column facing the inside of the centrifuge,
   ∘ Add 1st strand reaction to the center of the column ∘ Spin 2 min at 1,000 xG at RT
   ∘ Collect the flow-through solution (purified cDNA) and discard the column

### Second strand synthesis

1) Prepare reaction as follow:

| | **Stock conc.** | **Volume, ul** |
|---|---|---|
| **Library (add water to vol.)** | | 114 |
| **5X First strand buffer** | 5 X | 30 |
| **dNTP mix** | 10 mM each | 1 |
| **E.coli DNA polymerase I** | 10 U/ul | 4 |
| **E.coli RNase H** | 5U/ul | 1 |

2) Incubate 2h at 16C
3) Add 10µl 0.5M EDTA to stop reaction

### DNA shearing and size selection

To make the biotinylated DNA suitable for high-throughput sequencing, the DNA must be sheared to a size of 300-500 base pairs
1) Covaris instrument LE220 using micro tubes volume 130ul and following parameters:

| Fill level | Volume, ul | Duty Cycle | PIP | Cycles/burst | Time,sec |
|---|---|---|---|---|---|
| 10 | 130 | 15 | 500 | 200 | 57 |

2) After sonication load 5 ul of 1:10 dilution of sheared material on 2%TBE gel to verify size of fragments

### Size selection using SPRI (AMPure XP) beads

**Warm AMPure XP beads to RT and mix to homogenize.** Gently shake AMPure XP bottle to resuspend magnetic beads, and take needed volume in another tube. Warm AMPure XP beads to RT.
1) Take 1ml of SPRI suspension and separate beads using magnet. Remove 0.7ml of the clear solution to a new tube and keep it as "SPRI solution".
2) Resuspend the beads in the remaining 0.3ml of the solution and label it as "3xconc. SPRI beads"
3) Bring volume of sheared DNA to 200ul with water (or measure the volume if it is bigger than 200ul)
4) Add exactly 0.55X volumes of 3Xconc. beads to reaction, mix by pipette 10 times and incubate for about 5 min at room temperature.
5) Place mixture on the magnetic station; wait for the solution to clear.
6) Transfer cleared solution to the new tubes, avoiding any beads.
7) Add 3Xconc. SPRI to achieve final volume of bead solution equivalent to 0.7x of the initial volume (take into account that the mix contains already SPRI solution) to the cleared product, mix by pipette 10xtimes and incubate about 5 min at RT.
8) Place mixture on the magnetic station; wait for the solution to clear.
9) Transfer cleared solution to the new tubes (store as backup), KEEP the beads.
10) Wash the beads with 170ul 0.7XSPRI Solution in water mix (100ul water +70ul SPRI solution). Mix well and claim beads with the magnet. Discard solution.
11) Wash beads with 700 ul 70% ethanol WITHOUT mixing (while keeping on the magnet).
12) Remove wash solution and repeat 1 more time. Remove last drops of ethanol.
13) Leave beads on magnet for about 5 min to allow remaining ethanol to evaporate
14) Add 150 ul 1x TLE or water to the each tube, gently mix well by pipette. Incubate for about 5 min and separate beads from solution.
15) Transfer solution to a new labeled tube. Elute beads once more with 150ul TLE After separation, add solution to previous fraction.
16) Quantify by Qubit High Sensitivity assay
17) Run 3ul of 1:10 dilution on a 2% gel to verify the size of the selected library.

### Biotin pull-down

In this section of the protocol, ligation junctions are purified from the DNA pool, allowing for efficient identification of interacting chromatin fragments by paired-end sequencing.

### Perform all steps in low-bind tubes

2x Binding Buffer(BB): 10mM Tris-HCl (pH 7.5); 1mM EDTA; 2M NaCl. For 10 ml mix:
0.1ml x 1M Tris-HCl (pH8)
0.02ml x 0.5M EDTA
4ml x 5M NaCl
5.88ml water

IX Tween Washing Buffer(TWB): 5mM Tris-HCl (pH 7.5); 0.5mM EDTA; 1M NaCl; 0.05% Tween 20.
5ml 2X BB
4.95ml water
50ul 10% Tween20
IX Binding buffer (1XBB)
5ml 2XBB
5ml water
1) Prepare beads for biotin pull-down by washing 150 µl resuspended magnetic streptavidin beads (Dynabeads MyOne Streptavidin C1 beads 10mg/ml (7-12x109/ml)) twice with 400 µl Tween Buffer
2) These and future washes consist of five steps:
   i. Add buffer to the beads
   ii. Transfer the mixture to a new tube
   iii. Pipet up/down to mix well
   iv. Reclaim the beads using a magnetic particle concentrator
   v. Remove the supernatant
3) Resuspend the beads in 300 µl 2x BB (2x BB: 10 mM Tris-HCl pH 8.0, 1 mM EDTA, 2 M NaCl) and combine with 300 µl Hi-C DNA (final concentration of binding buffer is IX).
4) Allow the biotin labeled Hi-C DNA to bind to the streptavidin beads by incubating the mixture at room temperature for 15 minutes with rotation.
5) Reclaim the DNA bound streptavidin beads with the magnetic particle concentrator, and remove the supernatant.
6) Wash the beads transferring the suspension to a fresh tube each time in the following order:
   a. 600 ul of Tween Wash buffer at 55C
   b. 600 µl 1x BB (5 mM Tris-HCl pH 8.0, 0.5 mM EDTA, 1 M NaCl) at 55C. Repeat once more.
   c. 100 µl 1x ligation buffer.

### End-repair

1) **To repair the sheared DNA ends and breaks,** add:

| | Volume, ul |
|---|---|
| IX ligation buffer (NEB) | 90 |
| T4 DNA polymerase 1 (3U/ul) | 5 |
| T4 PNK (10U/ul) | 5 |
| Klenow DNA polymerase (5U/ul) | 1 |
| 25mM dNTP | 1.2 |

2) Incubate for 30 minutes at room temperature.
3) Wash the beads when transferring the suspension to a fresh tube each time in the following order:
d. 600 ul of Tween Wash buffer at 55C
e. 600 µl 1x BB at 55C. Repeat once more.
f. 100 µl 1x NEB2

4) **Attach a dATP to the 3' ends** of the end-repaired DNA by adding: 100 µl mix of
90ul 1x NEB2 buffer and transfer the mixture to a new tube
5ul 10mM dATP
6ul Klenow exo minus, 5U/ul
5) Incubate the reaction for 30 minutes at 37°C.
6) Following incubation, separate beads from solution using magnet and transferring to a fresh tube each time wash as follow:
a. 600 ul of Tween Wash buffer at 55C
b. 600 µl 1x BB at 55C
c. 100 µl 1XQuick Ligation Reaction Buffer (NEB M2200)
d. Resuspend beads in
50ul 1X Quick Ligation Reaction Buffer (NEB M2200)
2ul DNA Quicklig Ligase (NEB M2200)
7) To **ligate Illumina adapters,** add to each suspension:
3ul of Illumina indexed adapters (record all sample-index combinations)
8) Mix thoroughly. Place samples at room temperature for 15 minutes.
9) Remove non-ligated adapters by reclaiming the Hi-C DNA bound beads and washing the beads as follows:
a. 800 ul of Tween Wash Buffer at 55C. Repeat wash.
b. 600 µl 1x BB at 55C
c. 600ul 1x NEB2. Repeat wash using 100 ul 1X NEB2.

10) After the last wash, resuspend the beads in 50 µl 1x NEBuffer 2 and transfer to a new tube.

### Final amplification and purification for sequencing

1) To determine the number of cycles necessary to generate enough PCR product for sequencing, set up four test PCR reactions with 6, 9, or 12 cycles. Alternatively libraries could be amplified with 8 and higher number (e.g. 16 cycles) and amplicons are run on a gel to confirm size and quality.

| | Library | Control |
|---|---|---|
| | 2.0 | 0.0 |
| primers | 0.6 PE primer mix | 0.6 PE primer mix |
| 2XPhusion | 25.0 | 25.0 |
| water | 22.4 | 24.4 |
| final volume | 50.0 | 50.0 |

2) Set up PCR reactions and split into four strips. Cycle for 6/9/12

| | | |
|---|---|---|
| 98°C | 30s | |
| 98°C | 20 s | |
| 55or 65°C* | 30 s | |
| 72°C | 30 s | cycle x 6/9/12 or 8/16 |
| 72°C | 7min | |
| 4°C | indefinite | |

Illumina indexed primers optimal annealing T is 55C. Standard Illumina PE primers require 65C.
Determine the optimal cycle number by running 10ul PCR reactions (+2ul 6x loading dye) on a 6% polyacrylamide gel in 0.5XTBE ensuring the absence of spurious bands and the presence of a smear between 400-600 base pairs, which is the length of the sheared products after ligation to the adapters. Stain with Sybr Green (7ul in 70ml water) for 10 min. If there is time constrain, this step could be substituted by running lower resolution fast gel.
3) Amplify the rest (48ul) of the Hi-C-library-bound streptavidin beads in a large-scale PCR with the optimal number of PCR cycles. For 200ul PCR reaction, split into 4 wells of 96 well plate

| | Library [ul] | Control[ul] |
|---|---|---|
| sample volume | 48.0 | 0.0 |
| primers | 10 PE primer mix | 2.5 PE primer mix |
| 2XPhusion | 100.0 | 25.0 |
| water | 42.0 | 22.5 |
| final volume | 200.0 | 50.0 |

4) Set up PCRs into 96 well plates or strips 4x 50ul reactions. Perform amplification using the same settings.
5) After amplification is complete, pool the PCR products from the separate wells. Wash wells with little TLE, add to combined volume .and reclaim the beads. Resuspend reclimed beads in 50 ul TLE and store at -20C as a backup. Measure total volume of PCR product. Keep 1% of the large scale PCR product separate to run on a gel.
6) Purify the remainder of the PCR product with 1Xvolume of 2x conc. Ampure SPRI beads. As follows:
a. Pipet 10x times up-down bead-pcr mixture and leave at room temp for 5 mins
b. Separate the beads with PCR product attached from the PCR primers by holding against a magnet for a few minutes.
c. After separation of beads, remove supernatant and wash beads once with 70% ethanol.

7) Elute with 100ul TLE (leave beads in) and purify again using 0.7x SPRI or SPRI solution (if previously used beads were not removed) to remove further any traces of short products (primer- and adapter- dimmers). Separate beads and discard supernatant.
8) Wash with 0.7X SPRI solution. Wash 2x with 70% ethanol while keeping beads on the magnet. DO NOT mix. After removing the last drops of ethanol, air dry beads for 5 min at room temperature holding the beads against the magnet.
9) Elute DNA twice with 25ul TLE combining clear solution into a new tube.
10) Load 1ul of purified product on a precast 6% polyacrylamide gel. Stain using Sybr green and take image ensuring the successful removal of the PCR primers. Alternatively, sample can be loaded on a DNA Bioanalyzer chip for a QC Flashgel could be used, but it is not as sensitive and this is last-resort alternative.
11) Quantify purified amplified library using Qubit HS assay. Concentration is expected to be higher than 2ng/ul.

### MiSeq QC run

- Thaw 300 cycle cartridge and Hybridization buffer. Do not start denaturing step until hybridization buffer is fully thawed (keep on ice after that)
- Dilute your library to 4 nM in 10 mM Tris pH 8 (TLE, EB or water could also be used). To calculate the molarity of library, use the following equation:
- sample in nM= (sample concentration in ng/ul) × 10^6average fragment size in bp × 650 Da
- To denature your library, thoroughly mix 5 ul 4nM library with 5 ul freshly diluted 0.1 N NaOH. Spin down briefly and incubate **for exactly 5 min** at room temp.
- Add 990ul ice-cold Hybridization Buffer. Mix well and keep on ice. Run 12 pmol dilution of the library (600ul library+400ul Hyb buffer) on MiSeq. If long fragments are present in the final library- load higher concentration on Miseq because the molarity estimation based on average 500-550bp size will not be accurate.

### Sequence with at least 240 cycles (120 paired end)

### Results of formaldehyde cross-linked chromatin ligation

Products of ligation of genomic RNA-DNA were detected using primers for 28S rRNA (forward) and flanking MseI site on Cox2 mitochondrial DNA (reverse) rRNA 28SF:
Cox2-Mse1R: GGAAGTTCTATTGGCAGAACG (SEQ ID NO: 1)
**qRT-PCR** of ligated chromatin in low reaction volume (50ul) and full-size proximity ligation (8ml)

### Ave. threshold cycles Cp

### Positive: 50ul ligation volume genomic (+ligase) 36.1

Negative: 50ul ligation volume genomic (no ligase) ND
Negative RT: 50ul ligation volume genomic (no ligase; no transcriptase) ND

### Positive: 8ml ligation volume genomic (+ligase) 37.7

Negative: 8ml ligation volume genomic (no ligase) ND
Negative RT: 8ml ligation volume genomic (no ligase; no transcriptase) ND

PCR products from positive reactions were isolated and Sanger sequenced proving that these are chimeric RNA-DNA products

Highlighted sequence aligns to 28SrRNA and the rest of the sequence matches Cox2 gene.
**>productl**
**>product2**

### Example 2

### Implementing the COOL-AID Protocol to Map XIST lincRNA and the X-Chromosome Inactivation Center (XIC) Interactoins

The COOL-AID protocol is a proximity ligation approach for mapping the interaction of RNA and DNA molecules that are in close proximity in the genome. Thus COOL-AID is used to analyze the XIST-XIC interactions. It is well established that XIST coating begins at the XIC (Lee and Jaenisch, 1997); the presence of this interaction can be assessed by qPCR on the COOL-AID library and will thus serve as a positive control. Several X-chromosome loci, such as the pseudoautosomal regions, are known to escape inactivation, and will serve as negative controls. Once these controls are satisfied, Sanger sequencing is performed on selected clones for the purpose of final validation. Then high-throughput Illumina sequencing of the COOL-AID library is employed. This will generate genome-wide maps of DNA-RNA interactions. The quality of these maps is assessed, checking for non-specific interactions and non-regulatory sources of high background, such as the proximity of mRNA transcripts to their genes. A likely noise-reduction step is normalization for RNA-accessibility, since not all bound RNAs will be equally susceptible to digestion via RNAse VI. This normalization can be accomplished efficiently by halting the COOL-AID protocol immediately before and after RNAse VI digestion, and comparing the results.

### Example 2

### Implementing the COOL-AID Protocol to Map RNA-DNA Interactions across ES Differentiation

The COOL-AID protocol, is applied to pluripotent mouse Embryonic Stem Cells before and after retinoic-acid-induced differentiation down a neural lineage. As ES cells differentiate, they undergo major changes in their gene expression programs, upregulating differentiation genes, downregulating pluripotency genes, and remodeling the chromatin landscape (Aiba et al., 2009; Ramalho-Santos et al., 2002). Approximately 10,000 genes, or half the genetic complement, exhibit marked changes in expression during this process; nearly 3,000 do so in a lineage-specific fashion. Extensive ChIP-Seq and RNA-Seq data exists for mES cells, allowing the inventors to generate a comprehensive catalog of ncRNAs (Mikkelsen et al., 2007). Finally, the regulatory network rewires extensively during this differentiation process, suggesting that significant changes in DNA-RNA binding may be taking place; focusing on these differential interactions will strengthen the inventors ability to determine which DNA-RNA interactions play a causal role in the differentiation process. Thus the mES/retinoic acid lineage provides an ideal system for monitoring the dynamics of RNA-DNA interaction changes during cell differentiation. mES Oct4-GFP reporter cells are grown and treated with retinoic acid across a 6 day time course. At each day we will FACs sort differentiated cells from the population based on GFP-negative status. COOL-AID is performed on each time point, probing each time point using Illumina sequencing and generating a map of RNA-DNA interactions across ES differentiation. Because a single lane on the Illumina platform can generate over 25 million paired-end sequences, it is expected that one lane will be sufficient to probe all RNA-DNA interactions. The findings will significantly expand the understanding of the role ncRNAs play in genome regulation. More specifically, the maps will allow for the determination of how chromatin state gets established during differentiation. This dataset will allow for the probing of where particular ncRNAs bind and the changes induced by these binding events. The correlation between the binding of each ncRNA and the expression of nearby genes is examined, allowing for the identification of both activating and repressive ncRNA regulators. Taking advantage of the extensive annotation of the mouse genome, one will be able to see whether particular ncRNAs are associated with genes in particular pathways. This dataset should allow the determination of where each ncRNA associates with DNA, and it will also allow for probing how this specificity is imparted.

In view of the many possible embodiments to which the principles of our invention may be applied, it should be recognized that illustrated embodiments are only examples of the invention and should not be considered a limitation on the scope of the invention. Rather, the scope of the invention is defined by the following claims. We therefore claim as our invention all that comes within the scope of these claims.

## Claims

1. A method for detecting spatial proximity relationships between RNA and DNA molecules in a sample, comprising:
in a provided sample comprising fragmented RNA and DNA, wherein the RNA and DNA have ends capable of joining to other fragmented DNA and RNA, respectively;
optionally, further fragmenting the RNA and DNA in the sample;
joining at least one end of the fragmented RNA to the end of at least one fragmented DNA, to create at least one joined replication-competent RNA-DNA hybrid molecule, wherein the join encodes the information about the proximity of the RNA and DNA in the sample;
optionally, ligating a RNA adaptor nucleic acid sequence and a DNA adaptor nucleic acid sequence to the respective RNA and DNA ends of the at least one joined replication competent RNA-DNA hybrid molecule to facilitate amplification and/or selection of the target join DNA molecule;
reverse transcribing the at least one joined RNA-DNA hybrid molecule to create least one target join DNA molecule that retains the information of the join;
optionally, amplifying the target join DNA molecule using primers that specifically hybridize to the RNA adaptor nucleic acid sequence and the DNA adaptor nucleic acid sequence; optionally introducing at least one capture moiety into the amplified strand of the at least one target join DNA molecule;
determining the sequence of the at least one target join DNA molecule, thereby detecting spatial proximity relationships between RNA and DNA molecules; and
optionally, correlating the sequence of one or more target joins with a disease state or an environmental condition.

2. The method of claim 1, wherein the nucleic acids are held in a fixed position relative to one another.

3. The method of claim 1 or 2, wherein determining the sequence of the at least one target join DNA molecule comprises DNA sequencing and/or PCR at the site of the join; and/or comprises using a probe that specifically binds to the at least one target join DNA molecule at the site of the join, and wherein the probe specifically hybridizes to the target join both 5' and 3' of the site of the join and spans the site of the join.

4. The method of any one of claims 1-3, wherein the RNA and/or the DNA are end repaired.

5. The method of any one of claims 1-4, wherein at least one capture moiety is introduced into the at least one target join DNA molecule.

6. The method of claim 5, further comprising capturing the at least one target join DNA molecule and/or amplified strand of the at least one target join DNA molecule via the one or more capture moieties; optionally, the one or more capture moieties is captured on a solid support; optionally, the one or more capture moieties is captured with a capture moiety specific binding agent that specifically binds to the one or more capture moieties; optionally, the capture moiety specific binding agent is attached to the solid support; optionally, the molecular capture moieties comprises biotin, which is linked to a nucleotide and the capture moiety specific binding agent is streptavidin.

7. The method of any one of claim 1-6, wherein the sample is acellular or of one or more cells.

8. The method of claim 7, further comprising lysing the cells, isolating nuclei from the one or more cells, and/or lysing nuclei from the one or more cells.

9. The method of any one of claims 1-8, further comprising synthesizing a probe to one or more identified target joins, wherein the probe specifically hybridizes to the target join both 5' and 3' of the site of the join and spans the site of the join.

10. A method for diagnosing a disease or condition, the method comprising: detecting, in a sample, one or more target joins that is indicative of disease or condition, wherein detection of the one or more target joins that is indicative of a disease or condition diagnoses the disease or condition, wherein the target join is detected using the method of any one of claims 1-9.

11. The method according to any one of claims 1-9, wherein the at least one end of the fragmented RNA is joined to the end of the at least one fragmented DNA using a ligase.

## Patentansprüche

1. Verfahren zum Nachweis von Beziehungen räumlicher Nähe zwischen RNA- und DNA-Molekülen in einer Probe, bei dem man:
in einer fragmentierte RNA und DNA umfassenden bereitgestellten Probe, bei der die RNA und DNA zur Verknüpfung mit anderer fragmentierter DNA beziehungsweise RNA fähige Enden aufweisen,
gegebenenfalls die RNA und DNA in der Probe weiter fragmentiert,
mindestens ein Ende der fragmentierten RNA mit dem Ende mindestens einer fragmentierten DNA verknüpft, unter Bildung mindestens eines verknüpften, replikationskompetenten RNA-DNA-Hybridmoleküls, wobei die Verknüpfung für die Information über die Nähe der RNA und DNA in der Probe codiert,
gegebenenfalls eine RNA-Adapternukleinsäuresequenz und eine DNA-Adapternukleinsäuresequenz mit den entsprechenden RNA- und DNA-Enden des mindestens einen verknüpften replikationskompetenten RNA-DNA-Hybridmoleküls zur Ermöglichung von Amplifikation und/oder Selektion des Zielverknüpfungs-DNA-Moleküls ligiert,
das mindestens eine verknüpfte RNA-DNA-Hybridmolekül unter Bildung mindestens eines Zielverknüpfungs-DNA-Moleküls, in dem die Information der Verknüpfung erhalten geblieben ist, revers transkribiert,
gegebenenfalls das Zielverknüpfungs-DNA-Molekül unter Verwendung von spezifisch mit der RNA-Adapternukleinsäuresequenz und der DNA-Adapternukleinsäuresequenz hybrierenden Primern amplifiziert, gegebenenfalls in den amplifizierten Strang des mindestens einen Zielverknüpfungs-DNA-Moleküls mindestens eine Fängergruppierung einschleust,
die Sequenz des mindestens einen Zielverknüpfungs-DNA-Moleküls bestimmt und dadurch Beziehungen von räumlicher Nähe zwischen RNA- und DNA-Molekülen nachweist, und
gegebenenfalls die Sequenz einer oder mehrerer Zielverknüpfungen mit einem Krankheitsstatus oder einer Umweltbedingung korreliert.

2. Verfahren nach Anspruch 1, wobei die Nukleinsäuren in einer festgelegten Position zueinander gehalten werden.

3. Verfahren nach Anspruch 1 oder 2, wobei die Bestimmung der Sequenz des mindestens einen Zielverknüpfungs-DNA-Moleküls DNA-Sequenzierung und/oder PCR an der Verknüpfungsstelle umfasst und/oder die Verwendung einer spezifisch an das mindestens eine Zielverknüpfungs-DNA-Molekül an der Verknüpfungsstelle bindenden Sonde umfasst, und wobei die Sonde spezifisch mit der Zielverknüpfung hybridisiert, sowohl 5' als auch 3' von der Verknüpfungsstelle, und die Verknüpfungsstelle überbrückt.

4. Verfahren nach einem der Ansprüche 1-3, wobei die RNA und/oder die DNA endenrepariert werden.

5. Verfahren nach einem der Ansprüche 1-4, wobei in das mindestens eine Zielverknüpfungs-DNA-Molekül mindestens eine Fängergruppierung eingeschleust wird.

6. Verfahren nach Anspruch 5, weiterhin umfassend das Einfangen des mindestens einen Zielverknüpfungs-DNA-Moleküls und/oder amplifizierten Strangs des mindestens einen Zielverknüpfungs-DNA-Moleküls durch die eine oder die mehreren Fängergruppierungen, wobei die eine oder die mehreren Fängergruppierungen gegebenenfalls an einen festen Träger gebunden sind, wobei die eine oder die mehreren Fängergruppierungen gegebenenfalls an ein fängergruppierungsspezifisches Bindungsmittel gebunden sind, das spezifisch an die eine oder die mehreren Fängergruppierungen bindet, wobei das fängergruppierungsspezifische Bindungsmittel gegebenenfalls an den festen Träger gebunden ist, wobei die molekularen Fängergruppierungen gegebenenfalls Biotin umfassen, das an ein Nukleotid gebunden ist, und wobei es sich bei dem fängergruppierungsspezifischen Bindungsmittel um Streptavidin handelt.

7. Verfahren nach einem der Ansprüche 1-6, wobei die Probe azellulär ist oder eine oder mehrere Zellen umfasst.

8. Verfahren nach Anspruch 7, bei dem man weiterhin die Zellen lysiert, Kerne aus der einen oder den mehreren Zellen isoliert und/oder Kerne aus der einen oder den mehreren Zellen lysiert.

9. Verfahren nach einem der Ansprüche 1-8, bei dem man weiterhin eine Sonde für eine oder mehrere identifizierte Zielverknüpfungen synthetisiert, wobei die Sonde spezifisch mit der Zielverknüpfung hybridisiert, sowohl 5' als auch 3' von der Verknüpfungsstelle, und die Verknüpfungsstelle überbrückt.

10. Verfahren zur Diagnose einer Krankheit bzw. eines Leidens, wobei das Verfahren Folgendes umfasst: den Nachweis, in einer Probe, von einer oder mehreren für die Krankheit bzw. das Leiden indikativen Zielverknüpfungen, wobei der Nachweis der einen oder der mehreren Zielverknüpfungen, die für eine Krankheit oder ein Leiden indikativ sind, die Krankheit bzw. das Leiden diagnostiziert, wobei die Zielverknüpfung unter Anwendung des Verfahrens nach einem der Ansprüche 1-9 detektiert wird.

11. Verfahren nach einem der Ansprüche 1-9, wobei das mindestens eine Ende der fragmentierten RNA unter Verwendung einer Ligase mit dem Ende der mindestens einen fragmentierten DNA verknüpft wird.

## Revendications

1. Procédé de détection de relations de proximité spatiale entre des molécules d'ARN et d'ADN dans un échantillon, comprenant :
dans un échantillon fourni comprenant de l'ARN et de l'ADN fragmenté, dans lequel l'ARN et l'ADN ont des extrémités capables de s'assembler à d'autres ADN et ARN fragmentés, respectivement ; facultativement, la fragmentation plus avant de l'ARN et l'ADN dans l'échantillon ;
l'assemblage d'au moins une extrémité de l'ARN fragmenté à l'extrémité d'au moins un ADN fragmenté, pour créer au moins une molécule hybride ARN-ADN compétente en réplication assemblée, dans lequel l'assemblage code pour les informations sur la proximité de l'ARN et l'ADN dans l'échantillon ;
facultativement, la ligature d'une séquence d'acide nucléique d'adaptateur d'ARN et d'une séquence d'acide nucléique d'adaptateur d'ADN aux extrémités respectives de l'ARN et l'ADN de l'au moins une molécule hybride ARN-ADN compétente en réplication assemblée afin de permettre l'amplification et/ou la sélection de la molécule d'ADN d'assemblage cible ;
la transcription inverse de l'au moins une molécule hybride ARN-ADN assemblée pour créer au moins une molécule d'ADN d'assemblage cible qui conserve les informations de l'assemblage ;
facultativement, l'amplification de la molécule d'ADN d'assemblage cible au moyen d'amorces qui s'hybrident spécifiquement à la séquence d'acide nucléique d'adaptateur d'ARN et à la séquence d'acide nucléique d'adaptateur d'ADN ; facultativement, l'introduction d'au moins un fragment de capture dans le brin amplifié de l'au moins une molécule d'ADN d'assemblage cible ;
la détermination de la séquence de l'au moins une molécule d'ADN d'assemblage cible, de façon à détecter des relations de proximité spatiale entre les molécules d'ARN et d'ADN ; et
facultativement, la corrélation de la séquence d'un ou plusieurs assemblages cibles avec un état pathologique ou une condition environnementale.

2. Procédé de la revendication 1, dans lequel les acides nucléiques sont maintenus dans une position fixe les uns par rapport aux autres.

3. Procédé de la revendication 1 ou 2, dans lequel la détermination de la séquence de l'au moins une molécule d'ADN d'assemblage cible comprend le séquençage d'ADN et/ou la PCR au site de l'assemblage ; et/ou comprend l'utilisation d'une sonde qui se lie spécifiquement à l'au moins une molécule d'ADN d'assemblage cible au site de l'assemblage, et dans lequel la sonde s'hybride spécifiquement à l'assemblage cible à la fois en 5' et en 3' du site de l'assemblage et couvre le site de l'assemblage.

4. Procédé de l'une quelconque des revendications 1 à 3, dans lequel l'ARN et/ou l'ADN sont réparés aux extrémités.

5. Procédé de l'une quelconque des revendications 1 à 4, dans lequel au moins un fragment de capture est introduit dans l'au moins une molécule d'ADN d'assemblage cible.

6. Procédé de la revendication 5, comprenant en outre la capture de l'au moins une molécule d'ADN d'assemblage cible et/ou brin amplifié de l'au moins une molécule d'ADN d'assemblage cible par l'intermédiaire des un ou plusieurs fragments de capture ; facultativement, les un ou plusieurs fragments de capture sont capturés sur un support solide ; facultativement, les un ou plusieurs fragments de capture sont capturés avec un agent de liaison de fragment spécifique de capture qui se lie spécifiquement aux un ou plusieurs fragments de capture ; facultativement, l'agent de liaison de fragment spécifique de capture est lié au support solide ; facultativement, les fragments de capture moléculaires comprennent de la biotine, qui est liée à un nucléotide et l'agent de liaison de fragment spécifique de capture est la streptavidine.

7. Procédé de l'une quelconque des revendications 1 à 6, dans lequel l'échantillon est acellulaire ou d'une ou plusieurs cellules.

8. Procédé de la revendication 7, comprenant en outre la lyse des cellules, l'isolement des noyaux des une ou plusieurs cellules, et/ou la lyse des noyaux des une ou plusieurs cellules.

9. Procédé de l'une quelconque des revendications 1 à 8, comprenant en outre la synthèse d'une sonde pour un ou plusieurs assemblages cibles identifiés, dans lequel la sonde s'hybride spécifiquement à l'assemblage cible à la fois en 5' et en 3' du site de l'assemblage et couvre le site de l'assemblage.

10. Procédé de diagnostic d'une maladie ou affection, le procédé comprenant : la détection, dans un échantillon, d'un ou plusieurs assemblages cibles qui sont indicatifs d'une maladie ou affection, dans lequel la détection des un ou plusieurs assemblages cibles qui sont indicatifs d'une maladie ou affection diagnostique la maladie ou affection, dans lequel l'assemblage cible est détecté au moyen du procédé de l'une quelconque des revendications 1 à 9.

11. Procédé selon l'une quelconque des revendications 1 à 9, dans lequel l'au moins une extrémité de l'ARN fragmenté est assemblée à l'extrémité de l'au moins un ADN fragmenté au moyen d'une ligase.


### SEQUENCE LISTING

<110> The Broad Institute, Inc President and Fellows of Harvard College Massachusetts Institute of Technology Lieberman Aiden, Erez Guttman, Mitchell Stamenova, Elena Lander, Eric
<120> METHODS FOR THE DETECTION OF DNA-RNA PROXIMITY
<130> BROD-0200WP
<150> US 61/793,783
   <151> 2013-03-15
<160> 3
<170> PatentIn version 3.5
<210> 1
   <211> 21
   <212> DNA
   <213> Homo sapiens
<400> 1
   ggaagttcta ttggcagaac g 21
<210> 2
   <211> 190
   <212> DNA
   <213> Homo sapiens
<400> 2
   ggagagggtg taaatctcgc gccgggccgt accctaaaac catagggcac caatgatact    60
   gaagctacga atatactgac tatgaagacc tatgctttga ttcatatata atcccaacaa   120
   acgacctaaa acctggtgaa ctacgactgc tagaagttga taaccaagtc gttctgccaa   180
   tagaacttcc                                                          190
<210> 3
   <211> 190
   <212> DNA
   <213> Homo sapiens
<400> 3
   ggagagggtg taaatctcgc gccgggccgt accctaaaac catagggcac caatgatact    60
   gaagctacga atatactgac tatgaagacc tatgctttga ttcatatata atcccaacaa   120
   acgacctaaa acctggtgaa ctacgactgc tagaagttga taaccaagtc gttctgccaa   180
   tagaacttcc                                                          190